(19) Europäisches Patentamt
European Patent Office
Office européen des brevets

(11) Veröffentlichungsnummer: **0 353 558 B1**

(12) # EUROPÄISCHE PATENTSCHRIFT

(45) Veröffentlichungstag der Patentschrift: **07.12.94**

(51) Int. Cl.5: **C07D 249/08**, C07D 233/60, A01N 43/653, A01N 43/50

(21) Anmeldenummer: **89113418.1**

(22) Anmeldetag: **21.07.89**

Die Akte enthält technische Angaben, die nach dem Eingang der Anmeldung eingereicht wurden und die nicht in dieser Patentschrift enthalten sind.

(54) **Halogenvinyl-azol-Derivate.**

(30) Priorität: **03.08.88 DE 3826344**
**22.02.89 DE 3905377**
**22.02.89 DE 3905378**

(43) Veröffentlichungstag der Anmeldung:
**07.02.90 Patentblatt 90/06**

(45) Bekanntmachung des Hinweises auf die Patenterteilung:
**07.12.94 Patentblatt 94/49**

(84) Benannte Vertragsstaaten:
**BE CH DE ES FR GB IT LI NL SE**

(56) Entgegenhaltungen:
**EP-A- 0 097 426**
**EP-A- 0 207 590**
**EP-A- 0 380 276**
**DE-A- 3 935 575**

(73) Patentinhaber: **BAYER AG**

**D-51368 Leverkusen (DE)**

(72) Erfinder: **Jautelat, Manfred, Dr.**
**Müllersbaum 28**
**D-5093 Burscheid (DE)**
Erfinder: **Berg, Dieter, Dr.**
**Gellertweg 27**
**D-5600 Wuppertal 1 (DE)**
Erfinder: **Dutzmann, Stefan, Dr.**
**Leinenweberweg 33**
**D-4000 Düsseldorf 13 (DE)**
Erfinder: **Brandes, Wilhelm, Dr.**
**Eichendorffstrasse 3**
**D-5653 Leichlingen (DE)**
Erfinder: **Hänssler, Gerd, Dr.**
**Am Arenzberg 58a**
**D-5090 Leverkusen 3 (DE)**
Erfinder: **Mauler, Astrid, Dr.**
**Ernst-Klein-Strasse 2**
**D-5653 Leichlingen 1 (DE)**
Erfinder: **Paulus, Wilfried, Dr.**
**Deswatinesstrasse 90**
**D-4150 Krefeld 1 (DE)**
Erfinder: **Frie, Monika, Dr.**
**Im alten Driesch 1**
**D-5068 Odenthal (DE)**

**EP 0 353 558 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Halogenvinyl-azol-Derivate, mehrere Verfahren zu deren Herstellung und deren Verwendung als Mikrobizide im Pflanzenschutz und im Materialschutz.

Es ist bereits bekannt geworden, daß bestimmte Hydroxyethyl-azol-Derivate, die eine Alkenyl-Gruppe enthalten, zur Bekämpfung von Pilzen geeignet sind (vgl. EP-A 0 207 590 und EP-A 0 257 822). Die Wirkung dieser Stoffe ist gut, läßt aber bei niedrigen Aufwandmengen in manchen Fallen zu wünschen übrig.

Weiterhin ist bereits bekannt geworden, daß sich zahlreiche Azolyl-Derivate zum Schutz von nichtlebenden organischen Substraten gegen Befall durch Mikroorganismen einsetzen lassen (vgl. DE-A 3 116 607). So können zum Beispiel 1-(4-Chlorphenoxy)-3,3-dimethyl-1-(1,2,4-triazol-1-yl)-butan-2-ol und 1-(2,4-Dichlorphenyl)-4,4-dimethyl-2-(1,2,4-triazol-1-yl)-pentan-3-ol zu dem genannten Zweck eingesetzt werden. Die Wirksamkeit dieser Stoffe ist im Materialschutz aber nicht immer ausreichend.

Aus der EP-A 0 097 425 und der EP-A 0 097 426 sind fungizid wirksame Azolyl-Derivate bekannt. Es werden aber keine Verbindungen beschrieben, in denen eine halogenierte Vinylgruppe am Carbinol-Kohlenstoffatom vorhanden ist.

In der erst nach den Prioritätsdaten der vorliegenden Anmeldung publizierten EP-A 0 380 276 werden substituierte Azole offenbart, in denen das zentrale Kohlenstoffatom sowohl einen gegebenenfalls substituierten Phenylrest als auch eine am terminalen Kohlenstoffatom dichlorierte Vinylgruppe trägt. Entsprechende Verbindungen, in denen das endständige Kohlenstoffatom der Vinylgruppe nur einfach chloriert ist oder neben dem Chloratom noch ein Brom- oder Iodatom enthält, werden jedoch nicht erwähnt.

Es wurden nun neue Halogenvinyl-azol-Derivate der Formel

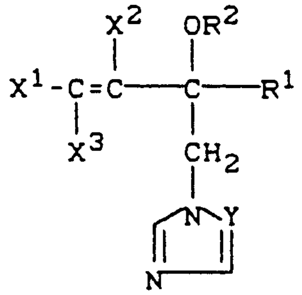

(I)

in welcher

- $R^1$ für geradkettiges oder verzweigtes Alkyl mit 1 bis 6 Kohlenstoffatomen steht, wobei jeder dieser Reste einfach bis dreifach, gleichartig oder verschieden substituiert sein kann durch Halogen, Cycloalkyl mit 3 bis 7 Kohlenstoffatomen, Phenyl und/oder Halogenphenyl, oder
- $R^1$ für Alkenyl mit 2 bis 6 Kohlenstoffatomen steht, wobei jeder dieser Reste einfach bis dreifach, gleichartig oder verschieden substituiert sein durch Halogen, Phenyl und/oder Halogenphenyl, oder
- $R^1$ für Cycloalkyl mit 3 bis 7 Kohlenstoffatomen steht, wobei jeder dieser Cycloalkylreste einfach bis dreifach, gleichartig oder verschieden substituiert sein kann durch Halogen und/oder Alkyl mit 1 bis 4 Kohlenstoffatomen, oder
- $R^1$ für einen gegebenenfalls benzanellierten fünf- oder sechsgliedrigen heteroaromatischen Rest mit 1 bis 3 Heteroatomen, steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, Formyl, Dialkoxymethyl mit 1 oder 2 Kohlenstoffatomen in jeder Alkoxygruppe,
  Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano,
- $R^2$ für Wasserstoff, Alkyl mit 1 bis 6 Kohlenstoffatomen, Alkenyl mit 3 bis 6 Kohlenstoffatomen, Acyl mit 1 bis 4 Kohlenstoffatomen oder Phenylalkyl mit 1 bis 4 Kohlenstoffatomen im Alkylteil steht,

$X^1$ für Fluor, Chlor, Brom oder Iod steht,
$X^2$ für Fluor, Chlor, Brom oder Iod steht,
$X^3$ für Wasserstoff, Chlor, Brom oder Iod steht und
Y für ein Stickstoffatom oder eine CH-Gruppe steht

oder auch

$R^1$ für Phenyl steht, das einfach bis dreifach, gleichartig oder verschieden substituiert sein kann durch Halogen, Alkyl mit 1 bis 4 Kohlenstofatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
sofern
$X^3$ für Wasserstoff, Brom oder Iod steht,

sowie deren Säureadditions-Salze und Metallsalz-Komplexe gefunden.

Die Verbindungen der Formel (I) enthalten ein asymmetrisch substituiertes Kohlenstoffatom und können deshalb in den beiden optischen Isomerenformen anfallen. Außerdem können die Stoffe der Formel (I) je nach der Stellung der Halogenatome an der Doppelbindung in zwei geometrischen Isomerenformen vorliegen. Die vorliegende Erfindung betrifft sowohl die Isomerengemische als auch die einzelnen Isomeren.

Weiterhin wurde gefunden, daß man Halogenvinyl-azol-Derivate der Formel (I) sowie deren Säureadditions-Salze und Metallsalz-Komplexe erhält, wenn man

a) Alkine der Formel

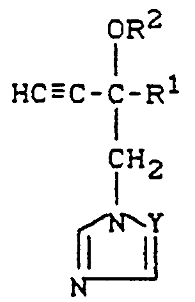

(II)

in welcher
$R^1$, $R^2$ und Y die oben angegebene Bedeutung haben,
mit Fluor, Chlor, Brom oder Iod oder mit diese Halogene liefernden Verbindungen in Gegenwart eines Verdünnungsmittels umsetzt,
oder

b) Alkene der Formel

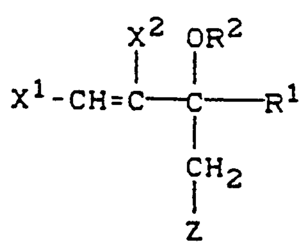

(III)

in welcher
$R^1$, $R^2$, $X^1$ und $X^2$ die oben angegebene Bedeutung haben und
Z für Chlor, Brom, Iod, Methylsulfonat oder p-Tolylsulfonat steht,
mit Azolen der Formel

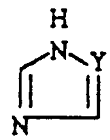

(IV)

in welcher

Y die oben angegebene Bedeutung hat,

in Gegenwart eines Säurebindemittels und in Gegenwart eines Verdünnungsmittels umsetzt,

oder

c) Alkine der Formel

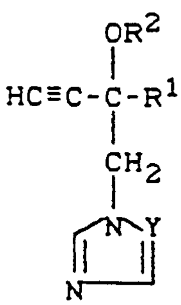

(II)

in welcher

$R^1$, $R^2$ und Y die oben angegebene Bedeutung haben,

in einer ersten Stufe mit Hypohalogeniten der Formel

$MOX^4$ (V)

in welcher

M für ein Natrium - oder halium ion steht und

$X^4$ für Chlor, Brom oder Iod steht,

in Gegenwart eines Verdünnungsmittels umsetzt und in einer zweiten Stufe die so erhaltenen Halogenalkine der Formel

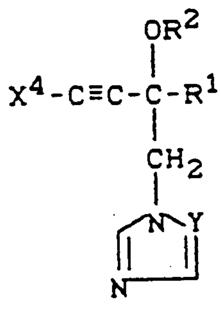

(VI)

in welcher

$R^1$, $R^2$, $R^4$ und Y die oben angegebene Bedeutung haben,

mit Fluor, Chlor, Brom oder Iod oder mit diese Halogene liefernden Verbindungen in Gegenwart eines Verdünnungsmittels umsetzt,

und gegebenenfalls anschließend an die so erhaltenen Verbindungen der Formel (I) eine Säure oder ein Metallsalz addiert.

Schließlich wurde gefunden, daß die neuen Halogenvinylazol-Derivate der Formel (I) sowie deren Säureadditions-Salze und Metallsalz-Komplexe starke mikrobizide Eigenschaften besitzen und sowohl im Pflanzenschutz als auch im Materialschutz eingesetzt werden können.

Überraschenderweise besitzen die erfindungsgemäßen Stoffe eine bessere fungizide Wirksamkeit im Pflanzenschutz als die konstitutionell ähnlichsten vorbekannten Verbindungen gleicher Wirkungsrichtung.

Außerdem eignen sich die erfindungsgemäßen Stoffe überraschenderweise auch besser zur Bekämpfung von unerwünschen Mikroorganismen im Materialschutz als 1-(4-Chlorophenoxy)-3,3-dimethyl-1-(1,2,4-triazol-1-yl)-butan-2-ol und 1-(2,4-Dichlorphenyl)-4,4-dimethyl-2-(1,2,4-triazol-1-yl)-pentan-3-ol, welche konstitutionell ähnliche, vorbekannte Wirkstoffe gleicher Wirkungsrichtung sind.

Die erfindungsgemäß Halogenvinyl-azol-Derivate sind durch die Formel (I) allgemein definiert. Bevorzugt verwendbar sind die Verbindungen der Formel (I), in denen

$R^1$ für Methyl, Ethyl, n-Propyl, Isopropyl, n-Butyl, tert.-Butyl, tert.-Pentyl, 1-Ethyl-1-methyl-propyl, 1,1-Dimethyl-pentyl, 1,1,2-Trimethylpropyl oder 1,1-Dimethyl-prop-2-enyl steht, wobei jeder dieser zuvorgenannten Reste einfach bis dreifach, gleichartig oder verschieden substituiert sein kann durch Fluor, Chlor, Brom, Phenyl, Chlorphenyl, Dichlorphenyl, Fluorphenyl und/oder Difluorphenyl, oder

$R^1$ für 1-Methyl-cyclohexyl, Cyclohexyl, 1-Chlor-cyclopropyl, 1-Methylcyclopropyl, Cyclopropyl, 1-Methylcyclopentyl, Cyclopentyl oder 1-Ethyl-cyclopentyl steht, oder

$R^1$ für Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl oder Benzimidazolyl steht, wobei jeder dieser Reste einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Hydroxymethyl, Hydroxyethyl, Hydroxyalkinyl mit 4 bis 6 Kohlenstoffatomen, Methoxycarbonyl, Ethoxycarbonyl, Methoximinomethyl, 1-Methoximino-ethyl, Nitro, Cyano, Formyl, Dimethoxymethyl, Acetyl und/oder Propionyl,

$R^2$ für Wasserstoff, Methyl, Ethyl, n-Propyl, Isopropyl, n-Butyl, Allyl, Formyl, Acetyl, Benzyl oder Phenethyl steht,

$X^1$ für Fluor, Chlor, Brom oder Iod steht,

$X^2$ für Fluor, Chlor, Brom oder Iod steht,

$X^3$ für Wasserstoff, Chlor, Brom oder Iod steht und

Y für ein Stickstoffatom oder eine CH-Gruppe steht,

oder auch

$R^1$ für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Chlordifluormethylthio, Methoxycarbonyl, Ethoxycarbonyl, Methoximinomethyl, 1-Methoximinoethyl, Nitro und/oder Cyano,

sofern

$X^3$ für Wasserstoff, Brom oder Iod steht.

Bevorzugte erfindungsgemäße Verbindungen sind auch Additionsprodukte aus Säuren und denjenigen Halogenvinyl-azol-Derivaten der Formel (I), in denen $R^1$, $R^2$, $X^1$, $X^2$, $X^3$ und Y diejenigen Bedeutungen haben, die für diese Substituenten als bevorzugt genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Halogenvinyl-azol-Derivaten der Formel (I), in denen $R^1$, $R^2$, $X^1$, $X^2$, $X^3$ und Y diejenigen Bedeutungen haben, die für diese Substituenten als bevorzugt genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für erfindungsgemäße Stoffe seien die in der folgenden Tabelle aufgeführten Halogenvinyl-azol-Derivate genannt.

Tabelle 1

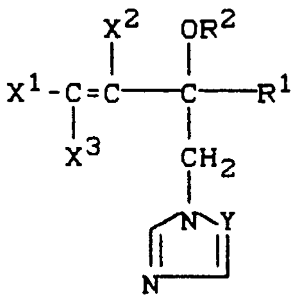

(I)

| $X^1$ | $X^2$ | $X^3$ | $R^1$ | $R^2$ | Y |
|---|---|---|---|---|---|
| J | J | H | $-C(CH_3)_3$ | H | N |
| F | F | H | $-C(CH_3)_3$ | H | N |
| J | J | H | $-C(CH_3)_3$ | H | CH |
| F | F | H | $-C(CH_3)_3$ | H | CH |
| Cl | Cl | H | $-C(CH_3)_3$ | $CH_3$ | N |
| Cl | Cl | H | $-C(CH_3)_3$ | $C_2H_5$ | N |
| Br | Br | H | $-C(CH_3)_3$ | $-CH_2-$ 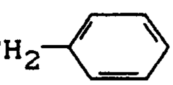 | N |
| Cl | Cl | H | $-C(CH_3)_3$ | $-CH_2-$ 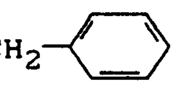 | N |
| Cl | Cl | H | 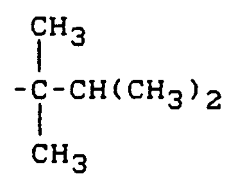 | H | N |
| Cl | Cl | H | 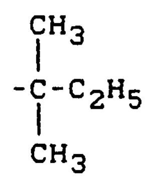 | H | N |

Tabelle 1 ( Fortsetzung)

| $X^1$ | $X^2$ | $X^3$ | $R^1$ | $R^2$ | Y |
|---|---|---|---|---|---|
| Cl | Cl | H | 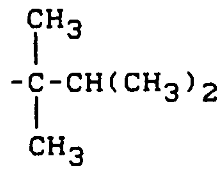 | H | N |
| Cl | Cl | H | 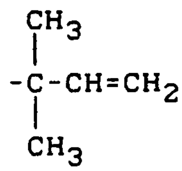 | H | N |
| Cl | Cl | H | 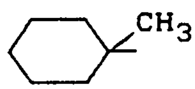 | H | N |
| Cl | Cl | H | 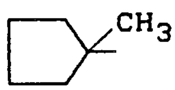 | H | N |
| Cl | Cl | H | 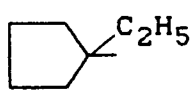 | H | N |
| Cl | Cl | H | 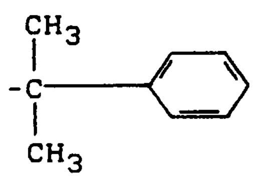 | H | N |
| Cl | Cl | H | 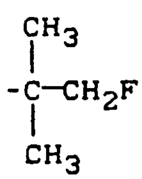 | H | N |
| Cl | Cl | H | 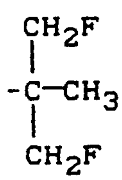 | H | N |

Tabelle 1 ( Fortsetzung)

| $X^1$ | $X^2$ | $X^3$ | $R^1$ | $R^2$ | Y |
|---|---|---|---|---|---|
| Cl | Cl | H | 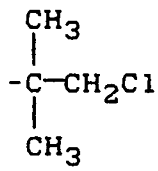 | H | N |
| Cl | Cl | H | 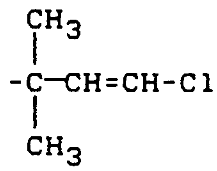 | H | N |
| Cl | Cl | H | 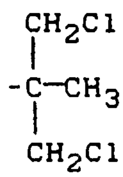 | H | N |
| Cl | Cl | H | 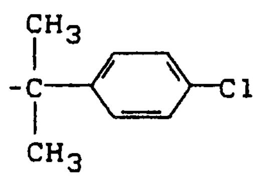 | H | N |
| Cl | Cl | H | 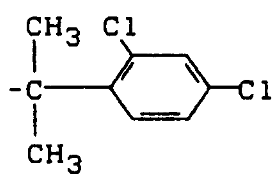 | H | N |
| Cl | Cl | H | 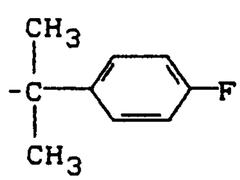 | H | N |
| Cl | Cl | H | 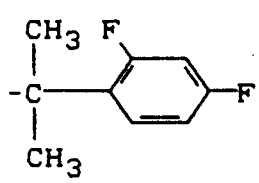 | H | N |

Tabelle 1 ( Fortsetzung)

| $X^1$ | $X^2$ | $X^3$ | $R^1$ | $R^2$ | Y |
|---|---|---|---|---|---|
| Cl | Cl | H | 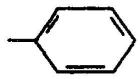 | H | N |
| Cl | Cl | H | 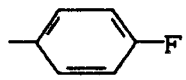 | H | N |
| Cl | Cl | H | 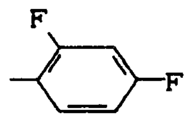 | H | N |
| Cl | Cl | H | 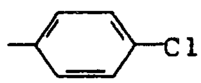 | H | N |
| Cl | Cl | H | 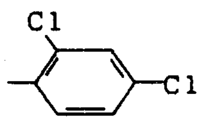 | H | N |
| Cl | Cl | H | 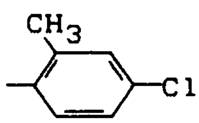 | H | N |
| Cl | Cl | H | 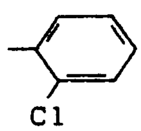 | H | N |
| Cl | Cl | H | 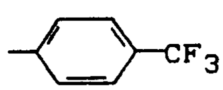 | H | N |
| Cl | Cl | H | 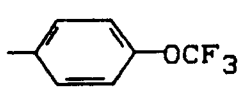 | H | N |

<u>Tabelle 1</u> ( Fortsetzung)

| $X^1$ | $X^2$ | $X^3$ | $R^1$ | $R^2$ | Y |
|---|---|---|---|---|---|
| Cl | Cl | H | 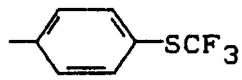 | H | N |
| Cl | Cl | H | 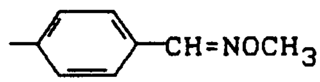 | H | N |
| Cl | Cl | H | 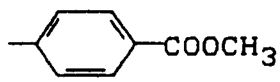 | H | N |
| Cl | Cl | H | 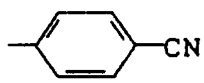 | H | N |
| Cl | Cl | H | 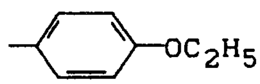 | H | N |
| Cl | Cl | H | 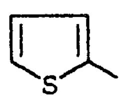 | H | N |
| Cl | Cl | H | 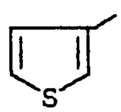 | H | N |
| Cl | Cl | H | 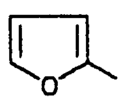 | H | N |
| Cl | Cl | H | 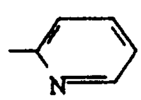 | H | N |
| Cl | Cl | H | 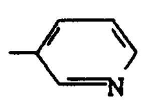 | H | N |

Tabelle 1 ( Fortsetzung)

| $X^1$ | $X^2$ | $X^3$ | $R^1$ | $R^2$ | Y |
|---|---|---|---|---|---|
| Cl | Cl | H | 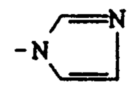 | H | N |
| Cl | Cl | H | 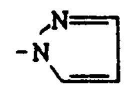 | H | N |
| Cl | Cl | H | 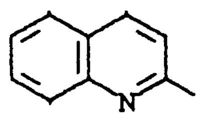 | H | N |
| Cl | Cl | Cl | $-C(CH_3)_3$ | $CH_3$ | N |
| Cl | Br | Br | $-C(CH_3)_3$ | $CH_3$ | N |
| Cl | Cl | Cl | 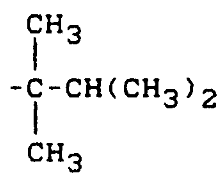 | H | N |
| Cl | Br | Br | 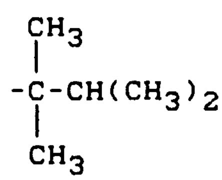 | H | N |
| Cl | Cl | Cl | 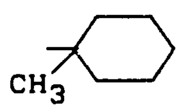 | H | N |
| Cl | Cl | Cl | 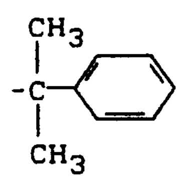 | H | N |
| Cl | Cl | Cl | 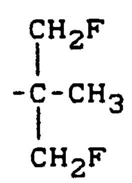 | H | N |

Verwendet man 4,4-Dimethyl-3-hydroxy-3-[(1,2,4-triazol-1-yl)-methyl]-pent-1-in als Ausgangsstoff und Chlorgas als Reaktionskomponente, so kann der Verlauf des erfindungsgemäßen Verfahrens (a) durch das folgende Formelschema veranschaulicht werden:

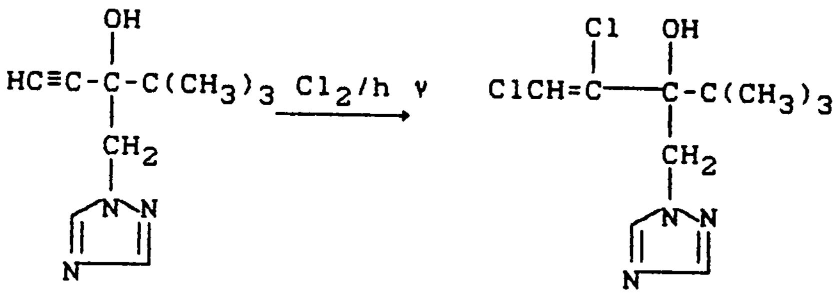


Verwendet man 1,2-Dichlor-3-hydroxy-3-chlormethyl-4,4-dimethyl-pent-1-en und 1,2,4-Triazol als Ausgangsstoffe, so kann der Verlauf des erfindungsgemäßen Verfahrens (b) durch das folgende Formelschema veranschaulicht werden:

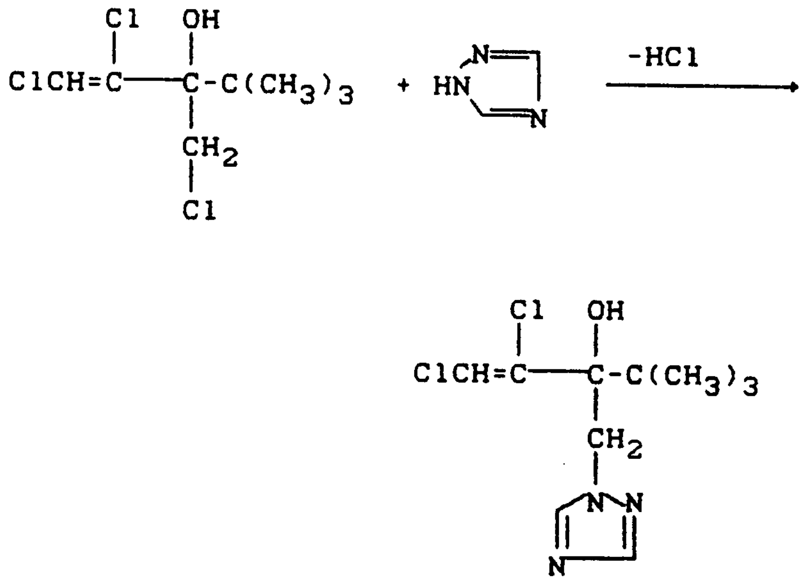


Verwendet man 4,4-Dimethyl-3-hydroxy-3-[(1,2,4-triazol-1-yl)-methyl]-pent-1-in als Ausgangsstoff und Natriumhypochlorit sowie Brom als Reaktionskomponenten, so kann der Verlauf des erfindungsgemäßen Verfahrens (c) durch das folgende Formelschema veranschaulicht werden:

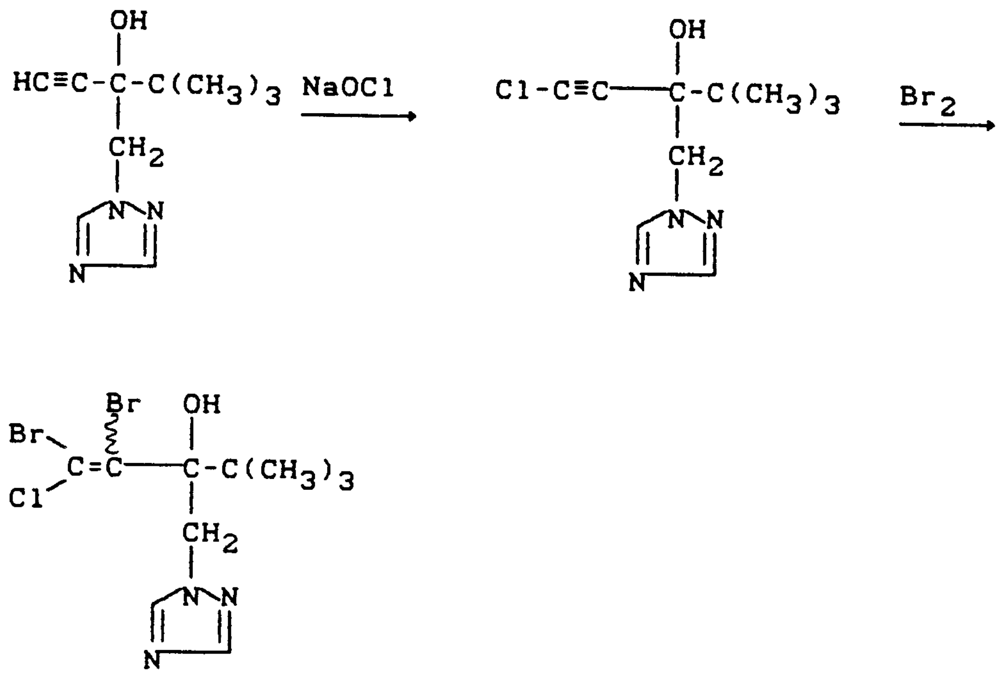


Die bei der Durchführung des erfindungsgemäßen Verfahrens (a) als Ausgangsstoffe benötigten Alkine sind durch die Formel (II) allgemein definiert. In dieser Formel haben $R^1$, $R^2$ und Y vorzugsweise diejenigen Bedeutungen, die bereits im Zusammenhang mit der Beschreibung der erfindungsgemäßen Stoffe der Formel (I) vorzugsweise für diese Reste genannte wurden.

Die Alkine der Formel (II) sind bisher noch nicht bekannt. Sie lassen sich herstellen, indem man

d) Azolyl-methyl-ketone der Formel

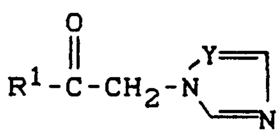 (VII)

in welcher

$R^1$ und Y die oben angegebene Bedeutung haben,

mit Acetylen-Salzen der Formel

HC≡CMe (VIII)

in welcher

Me für ein Äquivalent eines Metallkations steht,

in Gegenwart eines Verdünnungsmittels umsetzt und gegebenenfalls die dabei entstehenden Alkine der Formel

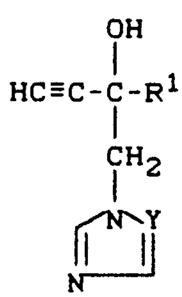 (IIa)

in welcher

$R^1$ und Y die oben angegebene Bedeutung haben,

mit starken Basen in Gegenwart eines Verdünnungsmittels umsetzt und die dabei entstehenden Alkoholate der Formel

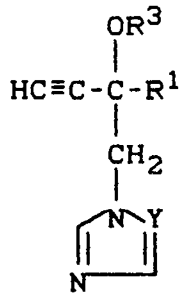

(IIb)

in welcher

$R^1$ und Y die oben angegebene Bedeutung haben,

und

$R^3$ für einen kationischen Rest einer Base steht, mit Halogen-Verbindungen der Formel

$R^4$-Hal (IX)

in welcher

$R^4$ für Alkyl, Alkenyl, Acyl oder Aralkyl steht und

Hal für Chlor, Brom oder Iod steht,

in Gegenwart eines Verdünnungsmittels umsetzt,

oder

e) Chlormethylketone der Formel

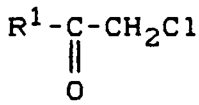

(X)

in welcher

$R^1$ die oben angegebene Bedeutung hat,

mit Acetylenen der Formel

$HC\equiv CR^5$ (XI)

in welcher

$R^5$ für Wasserstoff oder ein Äquivalent eines Metallkations steht,

gegebenenfalls in Gegenwart einer Base und in Gegenwart eines Verdünnungsmittels umsetzt und dann die dabei entstehenden Hydroxyalkine der Formel

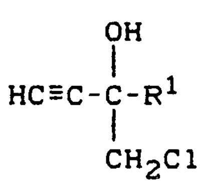

(XII)

in welcher

$R^1$ die oben angegebene Bedeutung hat,

mit Azolen der Formel

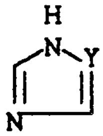

(IV)

in welcher

Y die oben angegebene Bedeutung hat,

in Gegenwart eines Säurebindemittels und in Gegenwart eines Verdünnungsmittels umsetzt und gegebenenfalls die dabei entstehenden Alkine der Formel

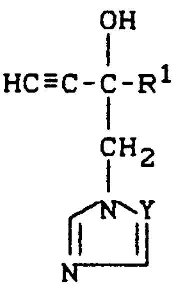

(IIa)

in welcher

$R^1$ und Y die oben angegebene Bedeutung haben,

gemäß Verfahren (d) weiter umsetzt.

Die bei der Durchführung des Verfahrens (d) als Ausgangsstoffe benötigten Azolyl-methyl-ketone sind durch die Formel (VII) allgemein definiert. In dieser Formel haben Y und $R^1$ vorzugsweise diejenigen Bedeutungen, die im Zusammenhang mit der Beschreibung der erfindungsgemäßen Stoffe der Formel (I) bereits vorzugsweise für diese Reste genannt wurden.

Die Azolyl-methyl-ketone der Formel (VII) sind bekannt oder lassen sich nach prinzipiell bekannten Verfahren in einfacher Weise herstellen (vgl. DE-A 2 431 407).

Die bei dem Verfahren (d) als Reaktionskomponenten benötigten Acetylen-Salze sind durch die Formel (VIII) allgemein definiert. In dieser Formel steht Me vorzugsweise für ein Lithiumkation oder für ein Äquivalent eines Cer (III)-Kations.

Die Acetylen-Salze der Formel (VIII) sind bekannt (vgl. Houben-Weyl, "Methoden der Organischen Chemie", Bd. V/2a, Seiten 509 ff., Georg Thieme Verlag, Stuttgart 1977 und Tetrahedron Letters 25, (1984) 4233).

Als Verdünnungsmittel können bei der Durchführung der ersten Stufe des Verfahrens (d) alle für derartige Umsetzungen üblichen inerten organischen Solventien verwendet werden. Vorzugsweise in Frage kommen Ether, wie Tetrahydrofuran oder Diethylether, und außerdem Kohlenwasserstoffe, wie n-Hexan.

Die Reaktionstemperaturen können bei der Durchführung der ersten Stufe des Verfahrens (d) innerhalb eines größeren Bereiches variiert werden. Im allgemeinen arbeitet man bei Temperaturen zwischen -78 °C und +30 °C, vorzugsweise bei Temperaturen zwischen -70 °C und +20 °C.

Bei der Durchführung des Verfahrens (d) arbeitet man ebenso wie bei der Durchführung der Verfahren (a), (b), (c) und (e) im allgemeinen unter Normaldruck.

Man geht bei der Durchführung der ersten Stufe des Verfahrens (d) im allgemeinen so vor, daß man zunächst die Acetylen-Salze herstellt und diese dann ohne vorherige Isolierung mit einer äquivalenten Menge, bzw. einem Über- oder Unterschuß an Azolyl-methyl-keton der Formel (VII) umsetzt. Die Aufarbeitung erfolgt nach üblichen Methoden. Im allgemeinen geht man so vor, daß man das Reaktionsgemisch zunächst mit einer wäßrigen Salz-Lösung, zum Beispiel Ammoniumchlorid-Lösung, versetzt, dann mehrfach mit einem in Wasser wenig löslichen organischen Solvens ausschüttelt und die vereinigten organischen Phasen nach dem Trocknen unter vermindertem Druck einengt.

Bei der Durchführung der zweiten Stufe des Verfahrens (d) erfolgt die Überführung der Alkine der Formel (IIa) in die entsprechenden Alkoholate, indem man mit geeigneten starken Basen, wie Alkalimetallamiden oder -hydriden, quarternären Ammonium-hydroxiden oder Phosphonium-hydroxiden in einem inerten Verdünnungsmittel, wie zum Beispiel Dioxan, bei Raumtemperatur umsetzt. Demgemäß steht $R^3$ in den Verbindungen der Formel (IIb) vorzugsweise für ein Alkalimetallkation, wie ein Natrium- oder Kaliumkation, oder für ein quarternäres Ammonium- oder Phosphoniumkation.

Die bei der Durchführung der dritten Stufe des Verfahrens (d) als Reaktionskomponenten benötigten Halogen-Verbindungen sind durch die Formel (IX) allgemein definiert. In dieser Formel steht $R^4$ vorzugsweise für die Bedeutung, die bereits im Zusammenhang mit der Beschreibung der erfindungsgemäßen Stoffe der Formel (I) für den Substituenten $R^2$ genannt wurden, mit Ausnahme der Bedeutung von Wasserstoff. Hal steht für Chlor, Brom oder Iod.

Die Halogenverbindungen der Formel (IX) sind bekannt oder lassen sich nach im Prinzip bekannten Methoden herstellen.

Als Verdünnungsmittel kommen bei der Durchführung der zweiten und dritten Stufe des Verfahrens (d) inerte organische Lösungsmittel in Frage. Vorzugsweise verwendbar sind Ether, wie Diethylether oder Dioxan; aromatische Kohlenwasserstoffe, wie Benzol; in einzelnen Fällen auch chlorierte Kohlenwasserstoffe, wie Chloroform, Methylenchlorid oder Tetrachlorkohlenstoff; sowie Hexamethylphosphorsäuretriamid.

Die Reaktionstemperaturen können bei der Durchführung der zweiten und dritten Stufe des Verfahrens (d) in einem größeren Bereich variiert werden. Im allgemeinen arbeitet man bei Temperaturen zwischen 0 °C und 120 °C, vorzugsweise zwischen 20 °C und 100 °C.

Bei der Durchführung der zweiten Stufe des Verfahrens (d) setzt man zunächst Alkine der Formel (IIa) mit starken Basen zu den entsprechenden Alkoholaten der Formel (IIb) um. In der folgenden dritten Stufe setzt man auf 1 Mol eines Alkoholates der Formel (IIb) vorzugsweise 1 bis 2 Mol Halogenverbindung der Formel (IX) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt.

In einer bevorzugten Ausführungsform wird in der zweiten und dritten Stufe des Verfahrens (d) zweckmäßigerweise so verfahren, daß man von einer Hydroxy-Verbindung der Formel (IIa) ausgeht, letztere in einem geeigneten organischen Lösungsmittel mittels Alkalimetall-hydrid oder Alkalimetall-amid in das Alkalimetallalkoholat überführt und letzteres ohne Isolierung sofort mit einer Halogenverbindung der Formel (IX) umsetzt, wobei unter Austritt von Alkalimetallhalogenid die Verbindungen der Formel (II) in einem Arbeitsgang erhalten werden.

Nach einer weiteren bevorzugten Ausführungsform werden zweckmäßigerweise die Herstellung der Alkoholate sowie die Umsetzung mit einer Halogenverbindung der Formel (IX) in einem Zweiphasensystem, wie beispielsweise wäßrige Natron- oder Kalilauge/Toluol oder Methylenchlorid, unter Zusatz von 0,01-1 Mol eines Phasen-Transfer-Katalysators, wie beispielsweise Ammonium- oder Phosphoniumverbindungen, durchgeführt, wobei in der organischen Phase oder an der Grenzfläche die Alkoholate mit den in der organischen Phase befindlichen Halogeniden umgesetzt werden.

Die bei der Durchführung des Verfahrens (e) als Ausgangsstoffe benötigten Chlormethylketone sind durch die Formel (X) allgemein definiert. In dieser Formel hat $R^1$ vorzugsweise diejenigen Bedeutungen, die bereits im Zusammenhang mit der Beschreibung der erfindungsgemäßen Stoffe der Formel (I) vorzugsweise für diesen Rest genannt wurden.

Die Chlormethylketone der Formel (X) sind bekannt oder lassen sich nach prinzipiell bekannten Methoden herstellen (vgl. DE-A 3 049 461).

Die bei dem Verfahren (e) als Reaktionskomponenten benötigten Acetylene sind durch die Formel (XI) allgemein definiert. In dieser Formel steht $R^5$ vorzugsweise für Wasserstoff, ein Lithium-kation oder ein Äquivalent eines Magnesium- oder Cer (III)-Kations.

Die Acetylene der Formel (XI) sind bekannt.

Als Basen kommen bei der Durchführung der ersten Stufe des Verfahrens (e) alle für derartige Umsetzungen üblichen starken Basen in Betracht. Vorzugsweise verwendbar sind Alkalimetallhydroxide, wie Kaliumhydroxid.

Als Verdünnungsmittel können bei der Durchführung der ersten Stufe des Verfahrens (e) alle für derartige Umsetzungen üblichen inerten organischen Solventien verwendet werden. Vorzugsweise in Frage kommen Ether, wie Tetrahydrofuran oder Diethylether.

Die Reaktionstemperaturen können bei der Durchführung der ersten Stufe des Verfahrens (e) innerhalb eines größeren Bereichs varriert werden. Im allgemeinen arbeitet man bei Temperaturen zwischen -78 °C und +50 °C, vorzugsweise zwischen -78 °C und +40 °C.

Bei der Durchführung der ersten Stufe des Verfahrens (e) geht man im allgemeinen so vor, daß man Chlormethylketone der Formel (X) und Acetylene der Formel (XI) in angenähert äquivalenten Mengen umsetzt. Es ist jedoch auch möglich, die eine oder andere Komponente in einem Überschuß zu verwenden. Die Aufarbeitung erfolgt nach üblichen Methoden. Die Hydroxyalkine der Formel (XII) können direkt weiter

umgesetzt werden mit Azolen der Formel (IV). Sie können aber auch zunächst in Oxirane überführt werden und dann mit Azolen der Formel (IV) umgesetzt werden.

Bei der Durchführung der zweiten Stufe des Verfahrens (e) kommen als Säurebindemittel alle üblichen Säureakzeptoren in Frage. Vorzugsweise verwendbar sind Alkalimetallcarbonate und Hydrogencarbonate, wie Natriumcarbonat, Kaliumcarbonat oder Natriumhydrogencarbonat, ferner tertiäre aliphatische oder aromatische Amine, wie Triethylamin, N,N-Dimethyl-cyclohexyl-amin, N,N-Dimethyl-benzylamin und Pyridin, und außerdem cyclische Amine, wie 1,5-Diaza-bicyclo[4.3.0]non-5-en (DBN), 1,8-Diaza-bicyclo[5.4.0]undec-7-en (DBU) und 1,4-Diazabicyclo [2.2.2]octan (DABCO).

Als Verdünnungsmittel kommen bei der Durchführung der zweiten Stufe des Verfahrens (e) alle inerten organischen Solventien in Betracht. Vorzugsweise verwendbar sind aliphatische und aromatische, gegebenenfalls halogenierte Kohlenwasserstoffe wie Pentan, Hexan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, sowie tert-Butylmethylether, Glykoldimethylether und Diglykoldimethylether, Tetrahydrofuran und Dioxan, Ketone wie Aceton, Methyl-ethyl-, Methyl-isopropyl- und Methyl-isobutylketon, Ester wie Essigsäuremethylester und -ethylester, Nitrile wie z.B. Acetonitril und Propionitril, und Pyridin.

Die Reaktionstemperaturen können auch bei der Durchführung der zweiten Stufe des Verfahrens (e) innerhalb eines größeren Bereiches variiert werden. Im allgemeinen arbeitet man bei Temperaturen zwischen 0 °C und 200 °C, vorzugsweise zwischen 20 °C und 150 °C.

Bei der Durchführung der zweiten Stufe des Verfahrens (e) geht man im allgemeinen so vor, daß man auf 1 Mol an Hydroxyalkin der Formel (XII) eine äquivalente Menge oder auch einen Überschuß an Azol der Formel (IV) sowie 2 bis 3 Mol an Säurebindemittel einsetzt. Die Aufarbeitung erfolgt nach üblichen Methoden. Die gegebenenfalls gewünschte weitere Umsetzung der Alkine der Formel (IIa) erfolgt bei dem Verfahren (e) in gleicher Weise wie bei dem Verfahren (d).

Als Halogene kommen bei der Durchführung des erfindungsgemäßen Verfahrens (a) vorzugsweise Fluor, Chlor, Brom und Iod als Reaktionskomponenten in Betracht, ferner gemischte Halogene wie Chlor(I)-fluorid, Brom(I)-fluorid, Iod(I)-fluorid, Brom(I)-chlorid, Jod(I)-chlorid oder Jod(I)-bromid (s. Methodicium Chimicium, F. Korte, Bd. 7, S. 842 (1976).

Als Halogen liefernde Verbindungen können beispielsweise Sulfurylchlorid, N-Bromsuccinimid mit Salzsäure, N-Chlorsuccinimid mit Bromwasserstoffsäure oder N-Chlorsucciniumid mit Fluorwasserstoff/Pyridin (s. Synthesis 1973, 780) verwendet werden.

Die Addition der Halogene an die Alkine der Formel (II) kann durch Einwirkung von Licht, durch Wärme, durch radikalbildende Substanzen, wie organische Peroxide, durch oberflächenaktive Stoffe, wie Aktivkohle, oder Metallsalze, wie Kupfer(II)-chlorid oder Eisen(III)-chlorid, begünstigt werden. Teilweise kann dadurch das Isomerenverhältnis (E/Z) beeinflußt weden (s. Houben-Weyl, Methoden der Org. Chemie, Bd V/3, S. 551 (1962)).

Als Verdünnungsmittel können bei der Durchführung des erfindungsgemäßen Verfahrens (a) alle für derartige Umsetzungen üblichen inerten organischen Solventien eingesetzt werden. Vorzugsweise verwendbar sind halogenierte aliphatische Kohlenwasserstoffe, wie Methylenchlorid, Chloroform und Tetrachlorkohlenstoff.

Die Temperaturen können bei der Durchführung des erfindungsgemäßen Verfahrens (a) innerhalb eines bestimmten Bereiches variiert werden. Im allgemeinen arbeitet man bei Temperaturen zwischen -10 °C und +120 °C, vorzugsweise zwischen -5 °C und +80 °C.

Bei der Durchführung des erfindungsgemäßen Verfahrens (a) setzt man auf 1 Mol an Alkin der Formel (II) im allgemeinen eine äquivalente Menge oder einen Überschuß an Halogen bzw. Halogen liefernder Verbindung ein. Die Aufarbeitung erfolgt nach üblichen Methoden. Im allgemeinen geht man so vor, daß man mit einem in Wasser wenig löslichen organischen Solvens verdünnt, mit Wasser wäscht und die organische Phase nach dem Trocknen einengt. Es ist jedoch auch möglich, das Reaktionsgemisch nach beendeter Umsetzung direkt durch Abziehen der flüchtigen Komponenten unter vermindertem Druck einzuengen. Die entstehenden Produkte können gegebenenfalls nach üblichen Methoden weiter gereinigt werden.

Die bei dem erfindungsgemäßen Verfahren (b) als Ausgangsstoffe benötigten Alkene sind durch die Formel (III) allgemein definiert. In dieser Formel haben $R^1$, $R^2$, $X^1$ und $X^2$ vorzugsweise diejenigen Bedeutungen, die bereits im Zusammenhang mit der Beschreibung der erfindungsgemäßen Stoffe der Formel (I) vorzugsweise für diese Stoffe genannt wurden. Z steht für Chlor, Brom, Iod, Methylsulfonat oder p-Tolylsulfonat.

Die Alkene der Formel (III) lassen sich nach üblichen Methoden herstellen. So erhält man zum Beispiel Alkene der Formel (III), indem man Hydroxyalkine der Formel (XII) mit Halogenen in Gegenwart eines

Verdünnungsmittels umsetzt. Die Reaktionsbedingungen entsprechen dabei denjenigen, die im Falle des erfindungsgemäßen Verfahrens (a) angewandt werden.

Als Verdünnungsmittel kommen bei der Durchführung des erfindungsgemäßen Verfahrens (b) alle üblichen inerten organischen Solventien in Betracht. Vorzugsweise verwendbar sind diejenigen Lösungsmittel, die bereits im Zusammenhang mit der Beschreibung der zweiten Stufe des Verfahrens (e) als bevorzugte Solventien genannt wurden.

Als Säurebindemittel kommen bei der Durchführung des erfindungsgemäßen Verfahrens (b) alle üblichen Säureakzeptoren in Betracht. Vorzugsweise verwendbar sind alle diejenigen Säurebindemittel, die bereits im Zusammenhang mit der Beschreibung der zweiten Stufe des Verfahrens (e) als bevorzugte Säureakzeptoren genannt wurden.

Die Reaktionstemperaturen können bei der Durchführung des erfindungsgemäßen Verfahrens (b) innerhalb eines größeren Bereiches variiert werden. Im allgemeinen arbeitet man bei Temperaturen zwischen 0°C und 150°C, vorzugsweise zwischen 20°C und 120°C.

Bei der Durchführung des erfindungsgemäßen Verfahrens (b) geht man im allgemeinen so vor, daß man auf 1 Mol an Alken der Formel (III) eine äquivalente Menge oder einen Überschuß an Azol der Formel (IV) sowie 2 bis 3 Mol an Säurebindemittel einsetzt. Die Aufarbeitung erfolgt nach üblichen Methoden.

Die bei der Durchführung des erfindungsgemäßen Verfahrens (c) als Reaktionskomponenten benötigten Hypohalogenite sind durch die Formel (V) allgemein definiert. In dieser Formel steht M für ein Natrium- oder Kalium-Ion, und $X^4$ steht für Chlor, Brom oder Iod.

Als Verdünnungsmitel können bei der Durchführung des erfindungsgemäßen Verfahrens (c) sowohl bei der Durchführung der ersten als auch der zweiten Stufe alle für derartige Umsetzungen üblichen inerten organischen Solventien eingesetzt werden. Vorzugsweise verwendbar sind halogenierte aliphatische Kohlenwasserstoffe, wie Methylenchlorid, Chloroform und Tetrachlorkohlenstoff.

Die Temperaturen können bei der Durchführung des erfindungsgemäßen Verfahrens (c) sowohl in der ersten als auch in der zweiten Stufe innerhalb eines bestimmten Bereiches variiert werden. Im allgemeinen arbeitet man bei Temperaturen zwischen -10°C und +120°C, vorzugsweise zwischen -5°C und +80°C.

Bei der Durchführung der ersten Stufe des erfindungsgemäßen Verfahrens (c) setzt man auf 1 Mol an Alkin der Formel (II) im allgemeinen einen Überschuß an Hypohalogenit ein. Bei der Durchführung der zweiten Stufe des erfindungsgemäßen Verfahrens (c) setzt man auf 1 Mol an Halogenalkin der Formel (VI) im allgemeinen eine äquivalente Menge oder einen Überschuß an Halogen ein. Die Aufarbeitung erfolgt sowohl bei der Durchführung der ersten als auch der zweiten Stufe nach üblichen Methoden.

Die nach den erfindungsgemäßen Verfahren erhältlichen Halogenvinyl-azol-Derivate der Formel (I) können in Säureadditions-Salze oder Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;

Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Getreide- und Reiskrankheiten, wie Pseudocercosporella, Erysiphe, Fusarium, Pyrenophora, Cochliobolus, Pyricularia und Pellicularia, sowie zur Bekämpfung von Gurkenmehltau und Apfelschorf und außerdem zur Bekämpfung von Botrytis im Obst-, Wein- und Gemüsebau.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nicht-lebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aurebasidium, wie Aureobasidium pullulans,
Sclerophoma, wie Sclerophoma pityophila,

Trichoderma, wie Trichoderma viride,
Escherichia, wie Escherichia coli,
Pseudomonas, wie Pseudomonas aeruginosa,
Staphylococcus, wie Staphylococcus aureus.

Im übrigen besitzen die erfindungsgemäßen Stoffe auch pflanzenwuchsregulierende Eigenschaften.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie, ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Pflanzenschutz im allgemeinen zwischen 0,1 und 95 Gewichtsprozent Wirkstoff, vorzugsweise zwischen 0,5 und 90 %.

Die erfindungsgemäßen Wirkstoffe können bei Verwendung im Pflanzenschutz in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren. Die Wirkstoffe können bei Verwendung im Pflanzenschutz als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Sattgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die zum Schutz technischer Materialien verwendeten mikrobiziden Mittel enthalten die Wirkstoffe im allgemeinen in einer Menge von 1 bis 95 Gew.-%, bevorzugt von 10 bis 75 Gew.-%.

Beim Einsatz im Materialschutz richten sich die Anwendungskonzentrationen an erfindungsgemäßen Wirkstoffen nach der Art und dem Vorkmmen der zu bekämpfenden Mikroorganismen sowie nach der Zusammen-setzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Auch beim Einsatz im Materialschutz können die erfindungsgemäßen Wirkstoffe in Mischung mit anderen bekannten Wirkstoffen angewendet werden.

Beispielsweise seien die folgenden Wirkstoffe genannt: Benzylalkoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Thiocyanatomethylthiobenzthiazol, Methylenbisthiocyanat, Phenolderivate wie 2-Phenylphenol, (2,2'-Dihydroxy-5,5'-dichlor)-diphenylmethan und 3-Methyl-4-chlor-phenol, Organo-Zinnverbindungen, N-Trihalogenmethylthio-Verbindungen, wie Folpet, Fluorfolpet, Dichlofluanid.

Die Herstellung und die Verwendung der erfindungsgemäßen Wirkstoffe werden durch die folgenden Beispiele veranschaulicht.

Herstellungsbeispiele

Beispiel 1

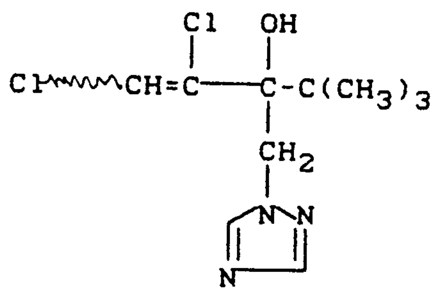

(I-1)

(B)

In eine Lösung von 3,86 g (20 mMol) 4,4-Dimethyl-3-hydroxy-3-[(1,2,4-triazol-1-yl)methyl]-1-pentin in 20 ml absolutem Methylenchlorid wird bei 0 bis 5 °C unter Bestrahlung durch eine 500 Watt-Lampe über einen Zeitraum von 6 Stunden ein Chlorgasstrom eingeleitet. Man rührt 15 Stunden bei Raumtemperatur nach, verdünnt das Reaktionsgemisch dann mit Methylenchlorid und schüttelt mit Wasser aus. Die organische Phase wird getrocknet und dann durch Abziehen des Lösungsmittels unter vermindertem Druck eingeengt. Man erhält 4,7 g (89% der Theorie) an 1,2-Dichlor-4,4-dimethyl-3-hydroxy-3-[(1,2,4-triazol-1-yl)-methyl]-1-penten in Form einer Festsubstanz vom Schmelzpunkt 49 - 52 °C. Bei diesem Stoff handelt es sich um ein Isomerengemisch A:B = 1:50.

NMR ($CDCl_3$):

(B) δ 1,15 (s, 9H) 4,3 (d, J = 14 Hz, 1H), 5,0 (d, J = 14 Hz, 1H), 6,68 (s, 1H), 7,95 (s, 1H), 8,25 (s, 1H)

Herstellung der Ausgangssubstanz:

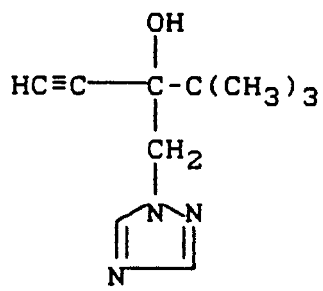

(II-1)

In 880 ml absolutem Tetrahydrofuran werden bei -70 °C 13 g (0,5 mol) Acetylen eingeleitet und durch Zutropfen von 200 ml (0,5 mol) Butyllithium in Hexan metalliert. Nach 30 Minuten wird eine Lösung von 78,5 g (0,47 mol) 3,3-Dimethyl-1-(1,2,4-triazolyl-1)-2-butanon in 150 ml absolutem Tetrahydrofuran bei -70 °C zugetropft. Das Reaktionsgemisch wird 2 Stunden bei -70 °C nachgerührt, dann aufgetaut und noch 2 Stunden bei 20 °C gerührt. Nach dem Verdünnen mit gesättigter, wäßriger Ammoniumchlorid-Lösung wird mehrfach mit Methylenchlorid ausgeschüttelt. Die vereinigten organischen Phasen werden getrocknet und dann unter vermindertem Druck eingeengt. Es verbleiben 74 g eines Produktes, das nach gaschromatographischer Analyse zu 38% aus 3,3-Dimethyl-1-(1,2,4-triazol-1-yl)-2-butanon und zu 57% aus 4,4-Dimethyl-3-hydroxy-3-[(1,2,4-triazol-1-yl)-methyl]-1-pentin besteht. Nach Umkristallisation aus Toluol erhält man reines 4,4-Dimethyl-3-hydroxy-[(1,2,4-triazol-1-yl)-methyl]-1-pentin vom Schmelzpunkt 129 - 131 °C.

NMR ($CDCl_3$): δ 1,2 (s, 9H), 2,35 (s, 1H), 3,75 (OH), 4,4 (AB, 2H), 8,0 (s, 1H), 8,25 (s, 1H).

Beispiel 2

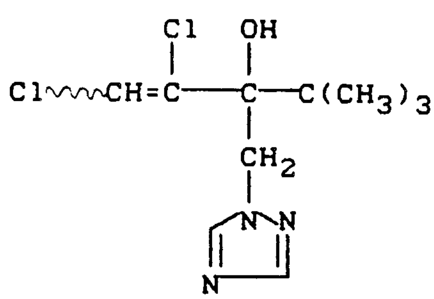

(I-2) (A)

25 g Aktivkohle werden als Granulat in 100 ml absolutem Methylenchlorid bei 0-5 °C vorgelegt. Dann werden 6,3 g (88 mMol) Chlorgas eingeleitet, anschließend 9,65 g (50 mMol) 4,4-Dimethyl-3-hydroxy-3-[-(1,2,4-triazol-1-yl)-methyl]-1-pentin in 100 ml absolutem Methylenchlorid zugetropft und 13 Stunden bei Raumtemperatur nachgerührt. Nach Filtration und Abziehen des Lösungsmittels bleiben 3,2 g 1,2-Dichlor-4,4-dimethyl-3-hydroxy-3-[(1,2,4-triazol-1-yl)-methyl]-1-penten als Isomerengemisch A:B = 25:1 vom Schmelzpunkt 59-62 °C zurück.

NMR ($CDCl_3$):

(A) δ 1,2 (s, 9H), 2,85 (OH), 4,3 (breites t, 1H), 5,0 (breites t, 1H), 6,35 (s, 1H), 8,05 (s, 1H), 8,4 (breites s, 1H)

Beispiel 3

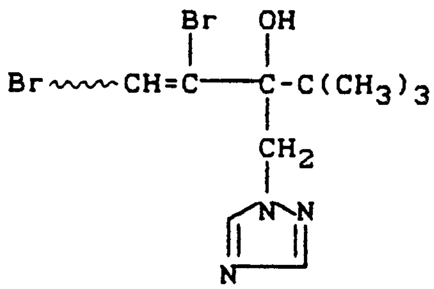

(I-3)

Zu einer Lösung von 3,86 g (20 mMol) 4,4-Dimethyl-3-hydroxy-3-[(1,2,4-triazol-1-yl)-methyl]-1-pentin in 30 ml absolutem Methylenchlorid wird bei 0 bis 5 °C unter Rühren eine Lösung von 6,4 g (40 mMol) Brom in 20 ml Methylenchlorid getropft. Das Reaktionsgemisch wird 5 Stunden bei 20 °C nachgerührt und dann durch Abziehen der flüchtigen Bestandteile unter vermindertem Druck eingeengt. Man erhält auf diese Weise 5,7 g (81% der Theorie) an 1,2-Dibrom-4,4-dimethyl-3-hydroxy-3-[(1,2,4-triazol-1-yl)-methyl]-1-penten in Form eines Öles. Bei diesem Stoff handelt es sich um ein Isomerengemisch A:B = 1:25.

NMR ($CDCl_3$) $\delta$:

(B) 1,25 (s, 9H), 4,4 (d, J = 15 Hz, 1H), 5,1 (d, J = 15 Hz, 1H), 6,7 (s, 1H), 8,2 (s, 1H), 8,9 (s, 1H).

Beispiel 4

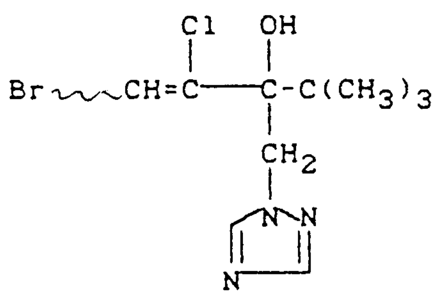

(I-4)

In die Lösung von 1,93 g (10 mMol) 4,4-Dimethyl-3-hydroxy-3-[(1,2,4-triazol-1-yl)-methyl]-1-pentin in 30 ml Methylenchlorid wird bei -20 °C Chlorwasserstoffgas bis zur Sättigung eingeleitet. Dann werden 1,8 g (20 mmol) N-Bromsuccinimid portionsweise zugegeben und 1 Std. bei 0 °C sowie 12 Std. bei Raumtemperatur nachgerührt. Im Vakuum werden HCl und Lösungsmittel abgezogen und der Rückstand mit Methylenchlorid und verd. Natronlauge aufgearbeitet. So erhält man 2,1 g (68 %. der Theorie) 1-Brom-2-chlor-4,4-dimethyl-3-hydroxy-3-((1,2,4-triazol-1-yl)-methyl]-1-penten als Isomerengemisch mit dem Schmelzpunkt 45-48 °C.

Nach den in den Beispielen 1 bis 4 angegebenen Methoden werden auch die in der folgenden Tabelle 2 aufgeführten Verbindungen hergestellt:

Tabelle 2

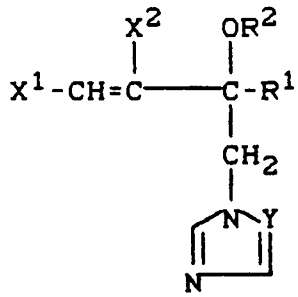

(Ia)

| Bei-spiel Nr. | Verb. Nr. | $X^1$ | $X^2$ | $R^1$ | $R^2$ | Y | Schmelz-punkt [°C] |
|---|---|---|---|---|---|---|---|
| 5 | I-5 | Cl | Cl | 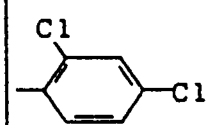 | H | N | 151-152 (Isomeren-gemisch) |
| 6 | I-6 | Br | Br | 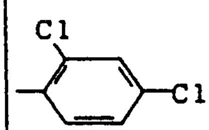 | H | N | 95 (Zers.) (Isomeren-gemisch) |
| 7 | I-7 | Br | Br | 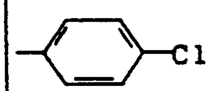 | H | N | 118-122 (Isomeren-gemisch) |
| 8 | I-8 | Cl | Cl | 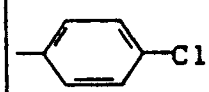 | H | N | 135-160 (Isomerenge-misch) |
| 9 | I-9 | Cl | Cl | $-C(CH_3)_3$ | $CH_3$ | N | Öl |
| 10 | I-10 | Cl | Cl | $-C(CH_3)_3$ | $COCH_3$ | N | 87- 92 |
| 11 | I-11 | Cl | Cl | 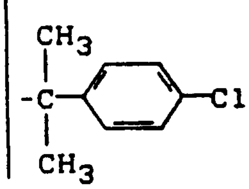 | H | N | Öl |

Tabelle 2 (Fortsetzung)

| Beispiel Nr. | Verb. Nr. | $X^1$ | $X^2$ | $R^1$ | $R^2$ | Y | Schmelzpunkt [°C] |
|---|---|---|---|---|---|---|---|
| 12 | I-12 | Cl | Cl | 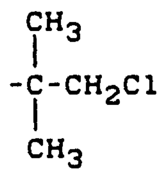 | H | N | Öl |
| 13 | I-13 | Br | Br | 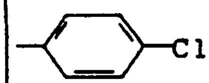 | H | CH | Öl |
| 14 | I-14 | Br | Br | 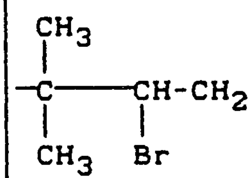 | H | N | Öl |
| 15 | I-15 | Br | Br | 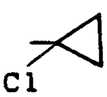 | H | N | 154-158 |
| 16 | I-16 | Cl | Cl | $-C(CH_3)_3$ | H | N | 53-56° (Isomerengemisch) |

Beispiel 17

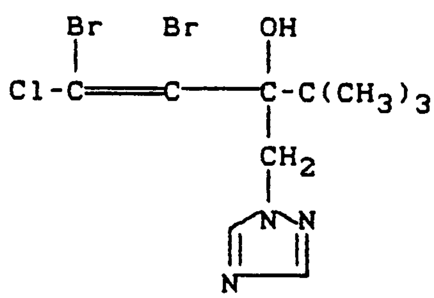

(I-17)

9,65 g (50 mMol) 4,4-Dimethyl-3-hydroxy-3-[(1,2,4-triazol-1-yl)-methyl]-1-pentin in 20 ml Methylenchlorid werden mit 400 ml (150 mMol) Natriumhypochlorit-Lösung 3 Tage bei Raumtemperatur gerührt. Dann wird das Reaktionsgemisch mit Methylenchlorid ausgeschüttelt. Nach dem Trocknen der organischen Phase und Abziehen des Lösungsmittels im Vakuum erhält man 9,8 g (43 mMol), 86 % 1-Chlor-4,4-dimethyl-3-hydroxy-3-[(1,2,4-triazol-1-yl)-methyl]-1-pentin vom Schmelzpunkt 88 °C.

NMR ($CDCl_3$): $\delta$ 1,15 (s, 3H), 4,4 (s, 2H) 8,0 (s, 1H), 8,3 (s, 1H)

Zu 2,3 g (10 mMol) 1-Chlor-4,4-dimethyl-3-hydroxy-3-[(1,2,4-triazol-1-yl)-methyl]-1-pentin in 20 ml absolutem Methylenchlorid werden bei 20 °C 1,6 g (10 mMol) Brom in 10 ml absolutem Methylenchlorid

gelöst getropft. Man rührt 5 Stunden bei Raumtemperatur nach und zieht dann das Lösungsmittel unter vermindertem Druck ab. Es verbleiben 3,8 g (100 % der Theorie) an 1-Chlor-1,2-dibrom-4,4-dimethyl-3-hydroxy-3-[(1,2,4-triazol-1-yl)-methyl-1-penten in Form einer Festsubstanz vom Schmelzpunkt 78 bis 81 °C.

Bei diesem Stoff handelt es sich um ein Isomerengemisch A:B = 1:3,6.

NMR ($CDCl_3$):

A) δ 1,25 (s, 9H), 4,4 (d, I = 14 Hz, 1H) 5,25 (d, I = 14 Hz, 1H), 5,6 (OH), 8,1 (s, 1H), 8,7 (s, 1H)

B) δ 1,25 (s, 9H), 4,6 (d, I = 14 Hz, 1H) 5,2 (d, I = 14 Hz, 1H), 5,6 (OH), 8,1 (s, 1H), 8,9 (s, 1H)

Nach der im Beispiel 17 angegebenen Methode werden auch die in der folgenden Tabelle 3 aufgeführten Verbindungen hergestellt.

**Tabelle 3**

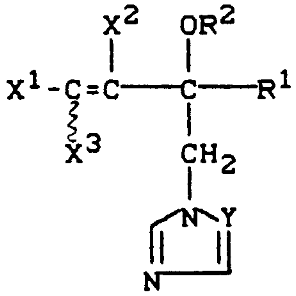

(I)

| Beispiel Nr. | Verb. Nr. | $X^1$ | $X^2$ | $X^3$ | $R^1$ | $R^2$ | Y | Schmelzpunkt (°C) |
|---|---|---|---|---|---|---|---|---|
| 18 | I-18 | Cl | Cl | Cl | $-C(CH_3)_3$ | H | N | Öl |
| 19 | I-20 | Cl | Cl | Cl | 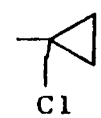 | H | N | Öl |

Verwendungsbeispiele

Beispiel A

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalles.

In diesem Test zeigen die erfindungsgemäßen Stoffe (I-1) und (I-3) eine sehr gute Wirksamkeit.

Beispiel B

Pellicularia-Test (Reis)

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25 °C und 100 % relativer Luftfeuchtigkeit aufgestellt.

5 bis 8 Tage nach der Inokulation erfolgt die Auswertung des Krankheitsbefalles.

In diesem Test zeigen die erfindungsgemäßen Stoffe (I-1) und (I-5) eine sehr gute Wirksamkeit.

Beispiel C

Botrytis-Test (Bohne)/protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit der angegebenen Menge Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

In deisem Test zeigt der erfindungsgemäße Stoff (I-1) eine sehr gute Wirksamkeit.

Beispiel D

Erysiphe-Test (Gerste)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die erfindungsgemäßen Stoffe (I-1) und (I-3) eine sehr gute Wirksamkeit.

Beispiel E

Leptosphaeria nodorum-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und Emulgator und verdünnt das Konzentrat mit Wasser und die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20 °C und 100% relative Luffeuchtigkeit in einer Inkubationskabine.

Die Pflanzen werden in einem Gewächshaus bei einer Temperatur von ca. 15 °C und einer relativen Luftfeuchtigkeit von ca. 80% aufgestellt.

10 Tage nach der Inokulation erfolgt die Auswertung.

In diesem Test zeigen die erfindungsgemäßen Stoffe (I-1) und (I-3) eine sehr gute Wirksamkeit.

Beispiel F

Pseudocercosporella herpotrichoides-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen an der Halmbasis mit Sporen von Pseudocercosporella herpotrichoides inokuliert.

Die Pflanzen werden in einem Gewächshaus bei einer Temperatur von ca. 10 °C und einer relativen Luftfeuchtigkeit von ca. 80 % aufgestellt.

21 Tage nach der Inokulation erfolgt die Auswertung.

In diesem Test zeigt der erfindungsgemäße Stoff (I-5) eine sehr gute Wirksamkeit.

Verwendungsbeispiele

In den folgenden Verwendungsbeispielen wurden die nachstehend angegebenen Verbindungen als Vergleichssubstanzen eingesetzt:

(A) 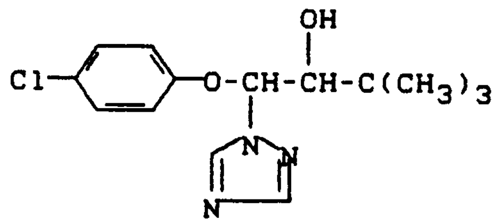

(B) 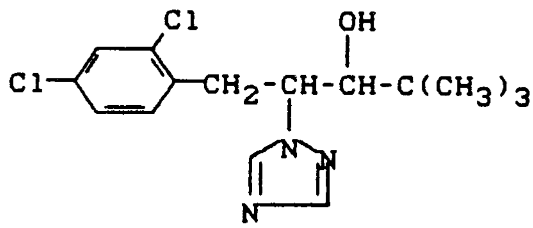

Beispiel G

Zum Nachweis der Wirksamkeit gegen Pilze werden die minimalen Hemm-Konzentrationen (MHK) von erfindungsgemäßen Wirkstoffen bestimmt:
Ein Agar, der aus Bierwürze und Pepton hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach zweiwöchiger Lagerung bei 28 °C und 60 bis 70 % relativer Luftfeuchtigkeit wird die MHK bestimmt. MHK ist die niedrigste Konzentration an Wirkstoff, bei der keinerlei Bewuchs durch die verwendete Mikrobenart erfolgt; sie ist in der nachstehenden Tabelle G angegeben.

Tabelle G

| MHK's in mg/l bei der Einwirkung von Substanzen auf Pilze | | | | | | | | |
|---|---|---|---|---|---|---|---|---|
| Testorganismen | Erfindungsgemäße Substanzen | | | | | | Vergleichssubstanzen | |
| | I-3 | I-1 | I-16 | I-17 | I-18 | I-12 | (A) | (B) |
| Alternaria tenuis | 35 | 50 | 35 | 500 | 100 | 20 | >1000 | >5000 |
| Aspergillus niger | 75 | 7,5 | 7,5 | 50 | <20 | 5 | >5000 | 5000 |
| Aureobasidiumpullulans | 35 | 5 | 7,5 | 50 | 10 | 5 | 1000 | >5000 |
| Chaetomium globosum | 100 | 5 | 7,5 | <20 | <20 | 2 | 90 | 5000 |
| Cladosporium cladosporioides | 100 | 20 | 50 | 200 | 50 | 20 | 500 | 1000 |
| Lentinus tigrinus | 20 | 5 | 5 | 20 | 7,5 | 10 | 750 | 500 |
| Penicillium glaucum | 50 | 200 | | 500 | 500 | 200 | 750 | 5000 |
| Sclerophoma pityophila | 20 | 0,5 | 1 | 5 | 1 | 0,5 | | |
| Trichoderma viride | 200 | 75 | 100 | 500 | 100 | 50 | | |

Beispiel H

Bestimmung der toxischen Grenzwerte (kg/m$^3$ Holz) erfindungsgemäßer Wirkstoffe für Coniophora puteana bzw. Polyporus versicolor auf Kiefern- bzw. Buchenholz

Die Bestimmung der toxischen Grenzwerte erfolgt in Anlehnung an die von H.P. Sutter, Int. Biodeterioration Bulletin 14 (3), 1978, Seiten 95 bis 99, beschriebene Methode.

Für die Tests werden jeweils frisch geschnittene, dünne Stirnholzstücke (Größe 40 x 40 mm, Dicke etwa 2 mm) im Vakuum mit Lösungen unterschiedlicher Wirkstoffkonzentration getränkt. Davon werden jeweils 5 gleichzeitig für einen mykologischen Test verwendet.

Die Menge des absorbierten Wirkstoffes wird aus der Lösungsmittelretention (die durch Wiegen des Holzstückchens vor und nach der Imprägnierung bestimmt wird), der Holzdichte und der Konzentration des Wirkstoffes in der zurückbleibenden Imprägnierlösung bestimmt.

Vor dem mykologischen Test werden die Prüflinge mit Propylenoxid sterilisiert und je 1 Prüfling in einer Petrischale in Kontakt mit dem voll entwickelten Mycel des Testpilzes auf Malzextraktagar gebracht. Nach 6 Wochen bei 21 bis 23 °C werden visuell die Toxizitätsgrenzen festgestellt.

In der folgenden Tabelle sind die Toxizitätsgrenzen (kg/m³ Holz) für erfindungsgemäße Substanzen angegeben; die Toxizitätsgrenzen zeigen die Konzentrationen, bei der das Holz noch angegriffen und bei der das Holz nicht mehr angegriffen wird.

Tabelle H

| Toxizitätsgrenzen (kg/m³ Holz) erfindungsgemäßer Wirkstoffe für holzzerstörende Pilze | | |
|---|---|---|
| Wirkstoff | Coniophora puteana auf Kiefernholz | Polyporus versicolor auf Buchenholz |
| I-3 | 0,71 - 1,42 | 0,21 - 0,68 |
| I-1 | 0,05 - 0,26 | < 0,02 |

**Patentansprüche**

**Patentansprüche für folgende Vertragsstaaten : BE, CH, DE, FR, GB, IT, LI, NL, SE**

**1.** Halogenvinyl-azol-Derivate der Formel

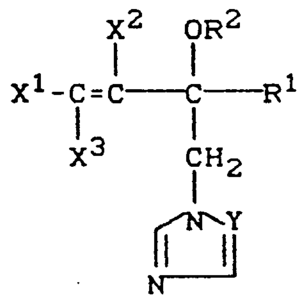

(I)

in welcher

$R^1$ für geradkettiges oder verzweigtes Alkyl mit 1 bis 6 Kohlenstoffatomen steht, wobei jeder dieser Reste einfach bis dreifach, gleichartig oder verschieden substituiert sein kann durch Halogen, Cycloalkyl mit 3 bis 7 Kohlenstoffatomen, Phenyl und/oder Halogenphenyl, oder

$R^1$ für Alkenyl mit 2 bis 6 Kohlenstoffatomen steht, wobei jeder dieser Reste einfach bis dreifach, gleichartig oder verschieden substituiert sein durch Halogen, Phenyl und/oder Halogenphenyl, oder

$R^1$ für Cycloalkyl mit 3 bis 7 Kohlenstoffatomen steht, wobei jeder dieser Cycloalkylreste einfach bis dreifach, gleichartig oder verschieden substituiert sein kann durch Halogen und/oder Alkyl mit 1 bis 4 Kohlenstoffatomen, oder

$R^1$ für einen gegebenenfalls benzanellierten fünf- oder sechsgliedrigen heteroaromatischen Rest mit 1 bis 3 Heteroatomen, steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, Formyl, Dialkoxymethyl mit 1 oder 2 Kohlenstoffatomen in jeder Alkoxygruppe,

Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano,

$R^2$ für Wasserstoff, Alkyl mit 1 bis 6 Kohlenstoffatomen, Alkenyl mit 3 bis 6 Kohlenstoffatomen, Acyl mit 1 bis 4 Kohlenstoffatomen oder Phenylalkyl mit 1 bis 4 Kohlenstoffatomen im Alkylteil steht,
$X^1$ für Fluor, Chlor, Brom oder Iod steht,
$X^2$ für Fluor, Chlor, Brom oder Iod steht,
$X^3$ für Wasserstoff, Chlor, Brom oder Iod steht und
Y für ein Stickstoffatom oder eine CH-Gruppe steht

oder auch

$R^1$ für Phenyl steht, das einfach bis dreifach, gleichartig oder verschieden substituiert sein kann durch Halogen, Alkyl mit 1 bis 4 Kohlenstofatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,
sofern
$X^3$ für Wasserstoff, Brom oder Iod steht,

sowie deren Säureadditions-Salze und Metallsalz-Komplexe.

**2.** Verfahren zur Herstellung von Halogenvinyl-azol-Derivaten der Formel (I) gemäß Anspruch 1 sowie von deren Säureadditions-Salzen und Metallsalz-Komplexen, dadurch gekennzeichnet, daß man

a) Alkine der Formel

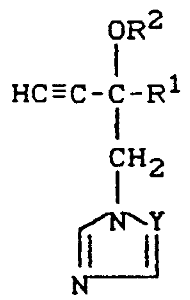

(II)

in welcher

$R^1$, $R^2$ und Y die oben angegebene Bedeutung haben,

mit Fluor, Chlor, Brom oder Iod oder mit diese Halogene liefernden Verbindungen in Gegenwart eines Verdünnungsmittels umsetzt,

oder

b) Alkene der Formel

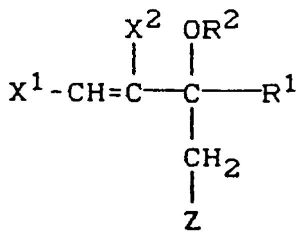

(III)

in welcher

$R^1$, $R^2$, $X^1$ und $X^2$ die oben angegebene Bedeutung haben und
Z für Chlor, Brom, Iod, Methylsulfonat oder p-Tolylsulfonat steht,

mit Azolen der Formel

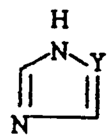

(IV)

in welcher

Y die oben angegebene Bedeutung hat,

in Gegenwart eines Säurebindemittels und in Gegenwart eines Verdünnungsmittels umsetzt, oder

c) Alkine der Formel

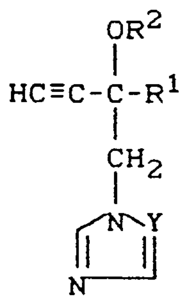

(II)

in welcher

$R^1$, $R^2$ und Y die oben angegebene Bedeutung haben,

in einer ersten Stufe mit Hypohalogeniten der Formel

$MOX^4$ (V)

in welcher

M für ein Natrium - oder halium ion steht und

$X^4$ für Chlor, Brom oder Iod steht,

in Gegenwart eines Verdünnungsmittels umsetzt und in einer zweiten Stufe die so erhaltenen Halogenalkine der Formel

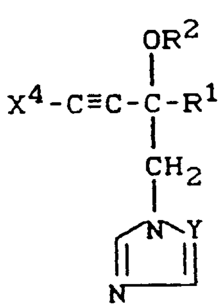

(VI)

in welcher

$R^1$, $R^2$, $X^4$ und Y die oben angegebene Bedeutung haben,

mit Fluor, Chlor, Brom oder Iod oder mit diese Halogene liefernden Verbindungen in Gegenwart eines Verdünnungsmittels umsetzt,

und gegebenenfalls anschließend an die so erhaltenen Verbindungen der Formel (I) eine Säure oder ein Metallsalz addiert.

**3.** Mikrobizide Mittel, gekennzeichnet durch einen Gehalt an mindestens einem Halogenvinyl-azol-Derivat der Formel (I) gemäß Anspruch 1 bzw. an einem Säureadditions-Salz oder Metallsalz-Komplex eines Halogenvinyl-azol-Derivates der Formel (I).

4. Verwendung von Halogenvinyl-azol-Derivaten der Formel (I) gemäß Anspruch 1 bzw. von deren Säureadditions-Salzen und Metallsalz-Komplexen als Mikrobizide im Pflanzenschutz und im Materialschutz.

5. Verfahren zur Bekämpfung von unerwünschten Mikroorganismen im Pflanzenschutz und im Materialschutz, dadurch gekennzeichnet, daß man Halogenvinyl-azol-Derivate der Formel (I) gemäß Anspruch 1 bzw. deren Säureadditions-Salze oder Metallsalz-Komplexe auf die Mikroorganismen und/oder deren Lebensraum ausbringt.

6. Verfahren zur Herstellung von mikrobiziden Mitteln, dadurch gekennzeichnet, daß man Hydroxyethyl-azol-Derivate der Formel (I) gemaß Anspruch 1 bzw. deren Säureadditions-Salze oder Metallsalz-Komplexe mit Streckmitteln und/oder oberflächenaktiven Stoffen vermischt.

**Patentansprüche für folgenden Vertragsstaat : ES**

1. Verfahren zur Herstellung von Halogenvinyl-azol-Derivaten der Formel

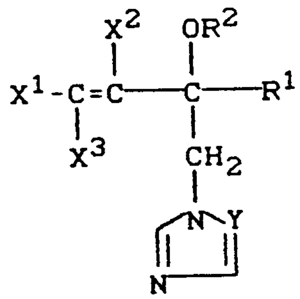

(I)

in welcher

$R^1$ für geradkettiges oder verzweigtes Alkyl mit 1 bis 6 Kohlenstoffatomen steht, wobei jeder dieser Reste einfach bis dreifach, gleichartig oder verschieden substituiert sein kann durch Halogen, Cycloalkyl mit 3 bis 7 Kohlenstoffatomen, Phenyl und/oder Halogenphenyl, oder

$R^1$ für Alkenyl mit 2 bis 6 Kohlenstoffatomen steht, wobei jeder dieser Reste einfach bis dreifach, gleichartig oder verschieden substituiert sein durch Halogen, Phenyl und/oder Halogenphenyl, oder

$R^1$ für Cycloalkyl mit 3 bis 7 Kohlenstoffatomen steht, wobei jeder dieser Cycloalkylreste einfach bis dreifach, gleichartig oder verschieden substituiert sein kann durch Halogen und/oder Alkyl mit 1 bis 4 Kohlenstoffatomen, oder

$R^1$ für einen gegebenenfalls benzanellierten fünf- oder sechsgliedrigen heteroaromatischen Rest mit 1 bis 3 Heteroatomen, steht, wobei jeder dieser Reste einfach bis dreifach, gleichartig oder verschieden substituiert sein kann durch Halogen, Alkyl mit 1 bis 4 Kohlenstoffatomen, Hydroxyalkyl mit 1 bis 4 Kohlenstoffatomen, Hydroxyalkinyl mit 3 bis 8 Kohlenstoffatomen, Alkoxy mit 1 oder 2 Kohlenstoffatomen, Alkylthio mit 1 oder 2 Kohlenstoffatomen, Halogenalkyl, Halogenalkoxy und Halogenalkylthlo mit jeweils 1 oder 2 Kohlenstoffatomen und 1 bis 5 gleichen oder verschiedenen Halogenatomen, Formyl, Dialkoxymethyl mit 1 oder 2 Kohlenstoffatomen in jeder Alkoxygruppe,
Acyl mit 2 bis 4 Kohlenstoffatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 3 Kohlenstoffatomen im Alkylteil, Nitro und/oder Cyano,

$R^2$ für Wasserstoff, Alkyl mit 1 bis 6 Kohlenstoffatomen, Alkenyl mit 3 bis 6 Kohlenstoffatomen, Acyl mit 1 bis 4 Kohlenstoffatomen oder Phenylalkyl mit 1 bis 4 Kohlenstoffatomen im Alkylteil steht,

$X^1$ für Fluor, Chlor, Brom oder lod steht,

$X^2$ für Fluor, Chlor, Brom oder lod steht,

$X^3$ für Wasserstoff, Chlor, Brom oder lod steht und

Y für ein Stickstoffatom oder eine CH-Gruppe steht

oder auch

$R^1$ für Phenyl steht, das einfach bis dreifach, gleichartig oder verschieden substituiert sein kann

durch Halogen, Alkyl mit 1 bis 4 Kohlenstofatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cycloalkyl mit 3 bis 7 Kohlenstoffatomen, Phenyl, Phenoxy, Alkoxycarbonyl mit 1 bis 4 Kohlenstoffatomen im Alkoxyteil, Alkoximinoalkyl mit 1 bis 4 Kohlenstoffatomen im Alkoxyteil und 1 bis 4 Kohlenstoffatomen im Alkylteil, Nitro und/oder Cyano,

sofern

$X^3$ für Wasserstoff, Brom oder Iod steht,

sowie von deren Säureadditions-Salzen und Metallsalz-Komplexen, dadurch gekennzeichnet, daß man

a) Alkine der Formel

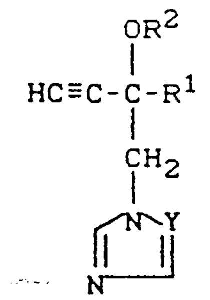

(II)

in welcher

$R^1$, $R^2$ und Y die oben angegebene Bedeutung haben,

mit Fluor, Chlor, Brom oder Iod oder mit diese Halogene liefernden Verbindungen in Gegenwart eines Verdünnungsmittels umsetzt,

oder

b) Alkene der Formel

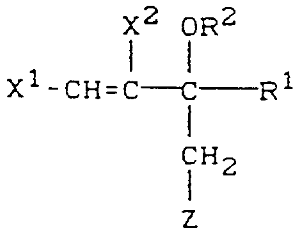

(III)

in welcher

$R^1$, $R^2$, $X^1$ und $X^2$ die oben angegebene Bedeutung haben und

Z für Chlor, Brom, Iod, Methylsulfonat oder p-Tolylsulfonat steht, mit Azolen der Formel

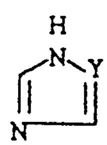

(IV)

in welcher

Y die oben angegebene Bedeutung hat,

in Gegenwart eines Säurebindemittels und in Gegenwart eines Verdünnungsmittels umsetzt,

oder

c) Alkine der Formel

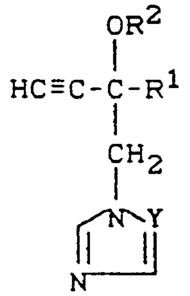

(II)

in welcher

$R^1$, $R^2$ und Y die oben angegebene Bedeutung haben,

in einer ersten Stufe mit Hypohalogeniten der Formel

$MOX^4$ (V)

in welcher

M für ein Natrium - oder halium ion steht und

$X^4$ für Chlor, Brom oder Iod steht,

in Gegenwart eines Verdünnungsmittels umsetzt und in einer zweiten Stufe die so erhaltenen Halogenalkine der Formel

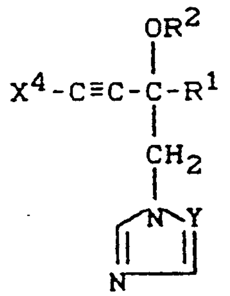

(VI)

in welcher

$R^1$, $R^2$ $X^4$ und Y die oben angegebene Bedeutung haben,

mit Fluor, Chlor, Brom oder Iod oder mit diese Halogene liefernden Verbindungen in Gegenwart eines Verdünnungsmittels umsetzt,

und gegebenenfalls anschließend an die so erhaltenen Verbindungen der Formel (I) eine Säure oder ein Metallsalz addiert.

**2.** Mikrobizide Mittel, gekennzeichnet durch einen Gehalt an mindestens einem Halogenvinyl-azol-Derivat der Formel (I) gemäß Anspruch 1 bzw. an einem Säureadditions-Salz oder Metallsalz-Komplex eines Halogenvinyl-azol-Derivates der Formel (I).

**3.** Verwendung von Halogenvinyl-azol-Derivaten der Formel (I) gemaß Anspruch 1 bzw. von deren Säureadditions-Salzen und Metallsalz-Komplexen als Mikrobizide im Pflanzenschutz und im Materialschutz.

**4.** Verfahren zur Bekämpfung von unerwünschten Mikroorganismen im Pflanzenschutz und im Materialschutz, dadurch gekennzeichnet, daß man Halogenvinyl-azol-Derivate der Formel (I) gemäß Anspruch 1 bzw. deren Säureadditions-Salze oder Metallsalz-Komplexe auf die Mikroorganismen und/oder deren Lebensraum ausbringt.

5. Verfahren zur Herstellung von mikrobiziden Mitteln, dadurch gekennzeichnet, daß man Hydroxyethyl-azol-Derivate der Formel (I) gemäß Anspruch 1 bzw. deren Säureadditions-Salze oder Metallsalz-Komplexe mit Streckmitteln und/oder oberflächenaktiven Stoffen vermischt.

## Claims

**Claims for the following Contracting States : BE, CH, DE, FR, GB, IT, LI, NL, SE**

1. Halogenovinyl-azole derivatives of the formula

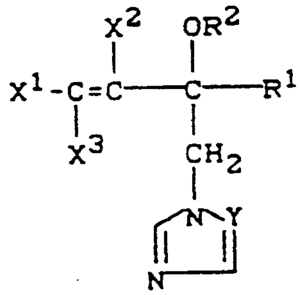

(I)

in which

$R^1$ represents straight-chain or branched alkyl having 1 to 6 carbon atoms, it being possible for each of these radicals to be substituted by one to three identical or different substituents from the group comprising halogen, cycloalkyl having 3 to 7 carbon atoms, phenyl and/or halogenophenyl, or

$R^1$ represents alkenyl having 2 to 6 carbon atoms, it being possible for each of these radicals to be substituted by one to three identical or different substituents from the group comprising halogen, phenyl and/or halogenophenyl, or

$R^1$ represents cycloalkyl having 3 to 7 carbon atoms, it being possible for each of these cycloalkyl radicals to be substituted by one to three identical or different substituents from the group comprising halogen and/or alkyl having 1 to 4 carbon atoms, or

$R^1$ represents an optionally benzo-fused five- or six-membered heteroaromatic radical having 1 to 3 heteroatoms, it being possible for each of these radicals to be substituted by one to three identical or different substituents from the group comprising halogen, alkyl having 1 to 4 carbon atoms, hydroxyalkyl having 1 to 4 carbon atoms, hydroxyalkinyl having 3 to 8 carbon atoms, alkoxy having 1 or 2 carbon atoms, alkylthio having 1 or 2 carbon atoms, halogenoalkyl, halogenoalkoxy and halogenoalkylthio having in each case 1 or 2 carbon atoms and 1 to 5 identical or different halogen atoms, formyl, dialkoxymethyl having 1 or 2 carbon atoms in each alkoxy group, acyl having 2 to 4 carbon atoms, alkoxycarbonyl having 1 to 4 carbon atoms in the alkoxy part, alkoximinoalkyl having 1 to 4 carbon atoms in the alkoxy part and 1 to 3 carbon atoms in the alkyl part, nitro and/or cyano,

$R^2$ represents hydrogen, alkyl having 1 to 6 carbon atoms, alkenyl having 3 to 6 carbon atoms, acyl having 1 to 4 carbon atoms or phenylalkyl having 1 to 4 carbon atoms in the alkyl part,

$X^1$ represents fluorine, chlorine, bromine or iodine,

$X^2$ represents fluorine, chlorine, bromine or iodine,

$X^3$ represents hydrogen, chlorine, bromine or iodine and

Y represents a nitrogen atom or a CH group,

or else

$R^1$ represents phenyl, which can be substituted by one to three identical or different substituents from the group comprising halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy part, alkoximinoalkyl having 1 to 4 carbon atoms in the alkoxy part and 1 to 4 carbon atoms in the alkyl part, nitro and/or cyano.

provided that

$X^3$ represents hydrogen, bromine or iodine,

and acid addition salts and metal salt complexes thereof.

**2.** Process for the preparation of halogenovinyl-azole derivatives of the formula (I) according to Claim 1 and of acid addition salts and metal salt complexes thereof, characterized in that

a) alkines of the formula

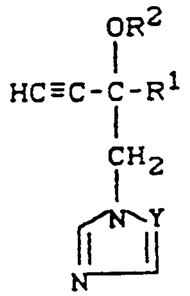

(II)

in which

$R^1$, $R^2$ and Y have the abovementioned meaning,

are reacted with fluorine, chlorine, bromine or iodine or with compounds which supply these halogens in the presence of a diluent,

or

b) alkenes of the formula

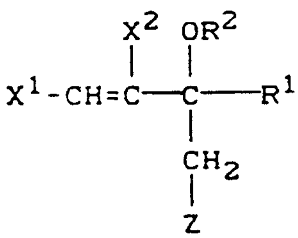

(III)

in which

$R^1$, $R^2$, $X^1$ and $X^2$ have the abovementioned meaning and

Z represents chlorine, bromine, iodine, methylsulphonate or p-tolylsulphonate,

are reacted with azoles of the formula

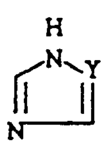

(IV)

in which

Y has the abovementioned meaning,

in the presence of an acid-binding agent and in the presence of a diluent,

or

c) alkines of the formula

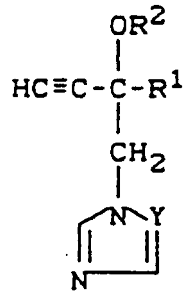

(II)

in which

$R^1$, $R^2$ and Y have the abovementioned meaning,

are reacted, in a first stage, with hypohalites of the formula

$MOX^4$ (V)

in which

M represents a sodium or potassium ion and

$X^4$ represents chlorine, bromine or iodine,

in the presence of a diluent, and, in a second stage, the halogenoalkines thus obtained, of the formula

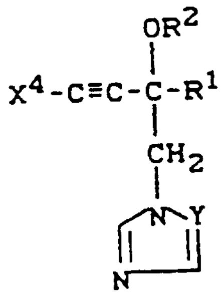

(VI)

in which

$R^1$, $R^2$, $X^4$ and Y have the abovementioned meaning,

are reacted with fluorine, chlorine, bromine or iodine or with compounds which supply these halogens in the presence of a diluent

and, if appropriate, an acid or a metal salt is then added onto the compounds of the formula (I) thus obtained.

**3.** Microbicidal agents, characterized in that they contain at least one halogenovinyl-azole derivative of the formula (I) according to Claim 1 or an acid addition salt or metal salt complex of a halogenovinyl-azole derivative of the formula (I).

**4.** Use of halogenovinyl-azole derivatives of the formula (I) according to Claim 1 or of acid addition salts and metal salt complexes thereof as microbicides in plant protection and in the preservation of materials.

**5.** Method of coating undesired microorganisms in plant protection and in the preservation of materials, characterized in that halogenovinyl-azole derivatives of the formula (I) according to Claim 1 or acid addition salts or metal salt complexes thereof are applied to the microorganisms and/or their environment.

6. Process for the preparation of microbicidal agents, characterized in that hydroxyethyl-azole derivatives of the formula (I) according to Claim 1 or acid addition salts or metal salt complexes thereof are mixed with extenders and/or surface-active substances.

**Claims for the following Contracting State : ES**

1. Process for the preparation of halogenovinyl-azole derivatives of the formula

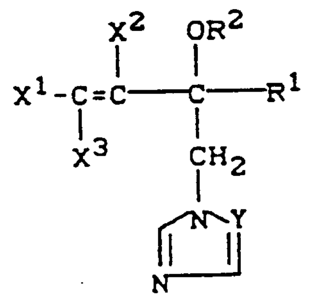

(I)

in which

$R^1$ represents straight-chain or branched alkyl having 1 to 6 carbon atoms, it being possible for each of these radicals to be substituted by one to three identical or different substituents from the group comprising halogen, cycloalkyl having 3 to 7 carbon atoms, phenyl and/or halogenophenyl, or

$R^1$ represents alkenyl having 2 to 6 carbon atoms, it being possible for each of these radicals to be substituted by one to three identical or different substituents from the group comprising halogen, phenyl and/or halogenophenyl, or

$R^1$ represents cycloalkyl having 3 to 7 carbon atoms, it being possible for each of these cycloalkyl radicals to be substituted by one to three identical or different substituents from the group comprising halogen and/or alkyl having 1 to 4 carbon atoms, or

$R^1$ represents an optionally benzo-fused five- or six-membered heteroaromatic radical having 1 to 3 heteroatoms, it being possible for each of these radicals to be substituted by one to three identical or different substituents from the group comprising halogen, alkyl having 1 to 4 carbon atoms, hydroxyalkyl having 1 to 4 carbon atoms, hydroxyalkinyl having 3 to 8 carbon atoms, alkoxy having 1 or 2 carbon atoms, alkylthio having 1 or 2 carbon atoms, halogenoalkyl, halogenoalkoxy and halogenoalkylthio having in each case 1 or 2 carbon atoms and 1 to 5 identical or different halogen atoms, formyl, dialkoxymethyl having 1 or 2 carbon atoms in each alkoxy group, acyl having 2 to 4 carbon atoms, alkoxycarbonyl having 1 to 4 carbon atoms in the alkoxy part, alkoximinoalkyl having 1 to 4 carbon atoms in the alkoxy part and 1 to 3 carbon atoms in the alkyl part, nitro and/or cyano,

$R^2$ represents hydrogen, alkyl having 1 to 6 carbon atoms, alkenyl having 3 to 6 carbon atoms, acyl having 1 to 4 carbon atoms or phenylalkyl having 1 to 4 carbon atoms in the alkyl part,

$X^1$ represents fluorine, chlorine, bromine or iodine,

$X^2$ represents fluorine, chlorine, bromine or iodine,

$X^3$ represents hydrogen, chlorine, bromine or iodine and

Y represents a nitrogen atom or a CH group,

or else

$R^1$ represents phenyl, which can be substituted by one to three identical or different substituents from the group comprising halogen, alkyl having 1 to 4 carbon atoms, alkoxy having 1 to 4 carbon atoms, alkylthio having 1 to 4 carbon atoms, halogenoalkyl having 1 or 2 carbon atoms and 1 to 5 identical or different halogen atoms, halogenoalkoxy having 1 or 2 carbon atoms and 1 to 5 identical or different halogen atoms, halogenoalkylthio having 1 or 2 carbon atoms and 1 to 5 identical or different halogen atoms, cycloalkyl having 3 to 7 carbon atoms, phenyl, phenoxy, alkoxycarbonyl having 1 to 4 carbon atoms in the alkoxy part, alkoximinoalkyl having 1 to 4 carbon atoms in the alkoxy part and 1 to 4 carbon atoms in the alkyl part, nitro and/or cyano.

<u>provided that</u>

$X^3$ represents hydrogen, bromine or iodine,

and of acid addition salts and metal salt complexes thereof characterized in that

a) alkines of the formula

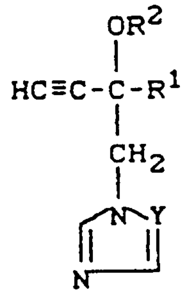

(II)

in which

$R^1$, $R^2$ and Y  have the abovementioned meaning,

are reacted with fluorine, chlorine, bromine or iodine or with compounds which supply these halogens in the presence of a diluent,

or

b) alkenes of the formula

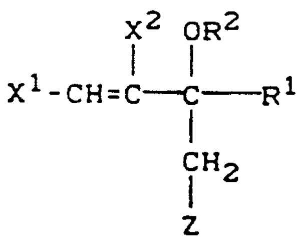

(III)

in which

$R^1$, $R^2$, $X^1$ and $X^2$  have the abovementioned meaning and

Z  represents chlorine, bromine, iodine, methylsulphonate or p-tolylsulphonate,

are reacted with azoles of the formula

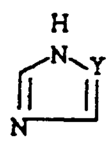

(IV)

in which

Y  has the abovementioned meaning,

in the presence of an acid-binding agent and in the presence of a diluent,

or

c) alkines of the formula

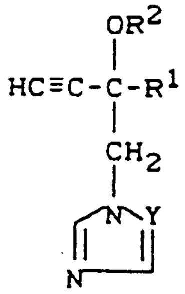

(II)

in which

$R^1$, $R^2$ and Y have the abovementioned meaning,

are reacted, in a first stage, with hypohalites of the formula

$MOX^4$ (V)

in which

M represents a sodium or potassium ion and

$X^4$ represents chlorine, bromine or iodine,

in the presence of a diluent, and, in a second stage, the halogenoalkines thus obtained, of the formula

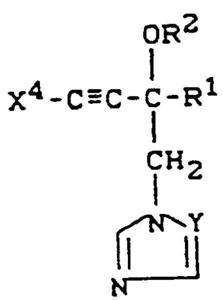

(VI)

in which

$R^1$, $R^2$, $X^4$ and Y have the abovementioned meaning,

are reacted with fluorine, chlorine, bromine or iodine or with compounds which supply these halogens in the presence of a diluent

and, if appropriate, an acid or a metal salt is then added onto the compounds of the formula (I) thus obtained.

**2.** Microbicidal agents, characterized in that they contain at least one halogenovinyl-azole derivative of the formula (I) according to Claim 1 or an acid addition salt or metal salt complex of a halogenovinyl-azole derivative of the formula (I).

**3.** Use of halogenovinyl-azole derivatives of the formula (I) according to Claim 1 or of acid addition salts and metal salt complexes thereof as microbicides in plant protection and in the preservation of materials.

**4.** Method of combating undesired microorganisms in plant protection and in the preservation of materials, characterized in that halogenovinyl-azole derivatives of the formula (I) according to Claim 1 or acid addition salts or metal salt complexes thereof are applied to the microorganisms and/or their environment.

5. Process for the preparation of microbicidal agents, characterized in that hydroxyethyl-azole derivatives of the formula (I) according to Claim 1 or acid addition salts or metal salt complexes thereof are mixed with extenders and/or surface-active substances.

## Revendications

**Revendications pour les Etats contractants suivants : BE, CH, DE, FR, GB, IT, LI, NL, SE**

1. Dérivés halogénovinyl-azoliques de formule

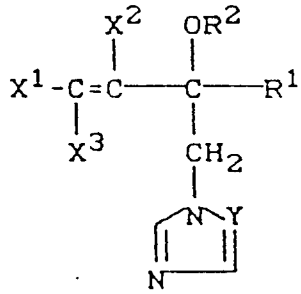

(I)

dans laquelle

$R^1$ est un reste alkyle linéaire ou ramifié ayant 1 à 6 atomes de carbone, chacun de ces restes pouvant être substitué une à trois fois identiques ou différentes par un radical halogéno, cycloalkyle ayant 3 à 7 atomes de carbone, phényle et/ou halogénophenyle, ou bien

$R^1$ est un reste alcényle ayant 2 à 6 atomes de carbone, chacun de ces restes pouvant être substitué une à trois fois identiques ou différentes par un radical halogéno, phényle et/ou halogénophényle, ou

$R^1$ est un reste cycloalkyle de 3 à 7 atomes de carbone, chacun de ces restes cycloalkyle pouvant être substitué une à trois fois identiques ou différentes par un radical halogéno et/ou alkyle ayant 1 à 4 atomes de carbone, ou bien

$R^1$ est un reste hétéroaromatique pentagonal ou hexagonal éventuellement condensé au benzène, ayant 1 à 3 hétéroatomes, chacun de ces restes pouvant être substitué une à trois fois identiques ou différentes par un radical halogéno, alkyle ayant 1 à 4 atomes de carbone, hydroxyalkyle ayant 1 à 4 atomes de carbone, hydroxyalcynyle ayant 3 à 8 atomes de carbone, alkoxy ayant 1 ou 2 atomes de carbone, alkylthio ayant 1 ou 2 atomes de carbone, halogénalkyle, halogénalkoxy et halogénalkylthio ayant chacun 1 ou 2 atomes de carbone et 1 à 5 atomes d'halogènes identiques ou différents, formyle, dialkoxyméthyle ayant 1 ou 2 atomes de carbone par groupe alkoxy,

acyle ayant 2 à 4 atomes de carbone, alkoxycarbonyle ayant 1 à 4 atomes de carbone dans la partie alkoxy, alkoximinoalkyle ayant 1 à 4 atomes de carbone dans la partie alkoxy et 1 à 3 atomes de carbone dans la partie alkyle, nitro et/ou cyano,

$R^2$ représente de l'hydrogène, un groupe alkyle ayant 1 à 6 atomes de carbone, alcényle ayant 3 à 6 atomes de carbone, acyle ayant 1 à 4 atomes de carbone ou phénylalkyle ayant 1 à 4 atomes de carbone dans la partie alkyle,

$X^1$ représente du fluor, du chlore, du brome ou de l'iode,

$X^2$ représente du fluor, du chlore, du brome ou de l'iode,

$X^3$ est de l'hydrogène, du chlore, du brome ou de l'iode et

Y est un atome d'azote ou un groupe CH,

ou aussi

$R^1$ est un groupe phényle qui peut être substitué une à trois fois identiques ou différentes par un halogène, un radical alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, cycloalkyle ayant 3 à 7 atomes de carbone, phényle, phénoxy, alkoxycarbonyle ayant 1 à 4 atomes de carbone dans la partie, alkoxy, alkoximinoalkyle ayant 1 à 4 atomes de carbone dans la partie alkoxy et 1 à 4 atomes de carbone dans la partie alkyle, nitro et/ou cyano,

dans la mesure où

$X^3$ est de l'hydrogène, du brome ou de l'iode,

ainsi que leurs sels d'addition d'acides et leurs complexes de sels métalliques.

**2.** Procédé de production de dérivés halogénovinyl-azoliques de formule (I) suivant la revendication 1, ainsi que de leurs sels d'addition d'acides et de leurs complexes de sels métalliques, caractérisé en ce que :

a) on fait réagir des alcynes de formule

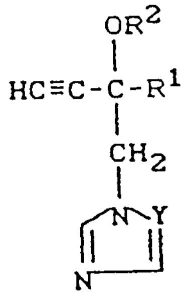

(II)

dans laquelle

$R^1$, $R^2$ et Y ont la définition indiquée ci-dessus,

avec du fluor, du chlore, du brome ou de l'iode ou avec des composés fournissant ces halogènes en présence d'un diluant,

ou bien

b) on fait réagir des alcènes de formule

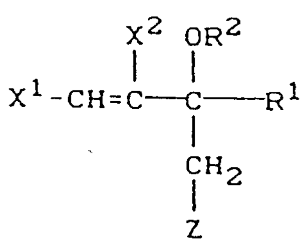

(III)

dans laquelle

$R^1$, $R^2$, $X^1$ et $X^2$ ont la définition indiquée ci-dessus et

Z représente du chlore, du brome, de l'iode, un groupe méthylsulfonate ou p-tolylsulfonate,

avec des azoles de formule

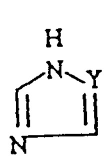

(IV)

dans laquelle

Y a la définition indiquée ci-dessus,

en présence d'un accepteur d'acide et en présence d'un diluant,

ou bien

c) on fait réagir des alcynes de formule

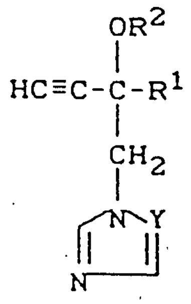

(II)

dans laquelle

$R^1$, $R^2$ et Y ont la définition indiquée ci-dessus,

dans une première étape avec des hypohalogénites de formule

$MOX^4$ (V)

dans laquelle

M est un ion de sodium ou de potassium et

$X^4$ représente du chlore, du brome ou de l'iode,

en présence d'un diluant et, dans une seconde étape, on fait réagir les halogénalcynes ainsi obtenues de formule

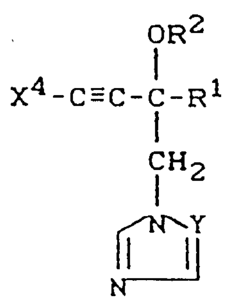

(VI)

dans laquelle

$R^1$, $R^2$, $X^4$ et Y ont la définition indiquée ci-dessus,

avec du fluor, du chlore, du brome ou de l'iode ou avec des composés fournissant ces halogènes, en présence d'un diluant,

puis, le cas échéant, on additionne un acide ou un sel métallique sur les composés de formule (I) ainsi obtenus.

**3.** Composition microbicide, caractérisée par une teneur en au moins un dérivé halogénovinyl-azolique de formule (I) suivant la revendication 1 ou en un sel d'addition d'acide ou un complexe de sel métallique d'un dérivé halogénovinyl-azolique de formule (I).

**4.** Utilisation de dérivés halogénovinyl-azoliques de formule (I) suivant la revendication 1 ou de leurs sels d'addition d'acides et de leurs complexes de sels métalliques comme microbicides dans la protection des plantes et dans la protection de matériaux.

**5.** Procédé pour combattre des micro-organismes indésirables dans la protection des plantes ou dans la protection des matériaux, caractérisé en ce qu'on fait agir des dérivés halogénovinyl-azoliques de formule (I) suivant la revendication 1 ou leurs sels d'addition d'acides ou leurs complexes de sels métalliques sur les micro-organismes et/ou sur leur milieu.

**6.** Procédé de préparation de compositions microbicides, caractérisé en ce qu'on mélange des dérivés hydroxyéthyl-azoliques de formule (I) suivant la revendication 1 ou leurs sels d'addition d'acides ou

leurs complexes de sels métalliques avec des diluants et/ou des agents tensio-actifs.

**Revendications pour l'Etat contractant suivant : ES**

**1.** Procédé de production de dérivés halogénovinyl-azoliques de formule

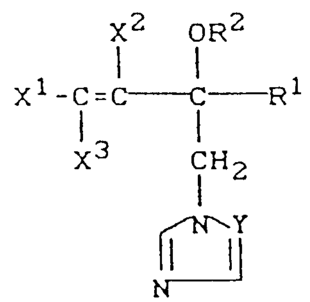

(I)

dans laquelle

$R^1$ est un reste alkyle linéaire ou ramifié ayant 1 à 6 atomes de carbone, chacun de ces restes pouvant être substitué une à trois fois identiques ou différentes par un radical halogéno, cycloalkyle ayant 3 à 7 atomes de carbone, phényle et/ou halogénophényle, ou bien

$R^1$ est un reste alcényle ayant 2 à 6 atomes de carbone, chacun de ces restes pouvant être substitué une à trois fois identiques ou différentes par un radical halogéno, phényle et/ou halogénophényle, ou

$R^1$ est un reste cycloalkyle de 3 à 7 atomes de carbone, chacun de ces restes cycloalkyle pouvant être substitué une à trois fois identiques ou différentes par un radical halogéno et/ou alkyle ayant 1 à 4 atomes de carbone, ou bien

$R^1$ est un reste hétéroaromatique pentagonal ou hexagonal éventuellement condensé au benzène, ayant 1 à 3 hétéroatomes, chacun de ces restes pouvant être substitué une à trois fois identiques ou différentes par un radical halogéno, alkyle ayant 1 à 4 atomes de carbone, hydroxyalkyle ayant 1 à 4 atomes de carbone, hydroxyalcynyle ayant 3 à 8 atomes de carbone, alkoxy ayant 1 ou 2 atomes de carbone, alkylthio ayant 1 ou 2 atomes de carbone, halogénalkyle, halogénalkoxy et halogénalkylthio ayant chacun 1 ou 2 atomes de carbone et 1 à 5 atomes d'halogènes identiques ou différents, formyle, dialkoxyméthyle ayant 1 ou 2 atomes de carbone par groupe alkoxy,
acyle ayant 2 à 4 atomes de carbone, alkoxycarbonyle ayant 1 à 4 atomes de carbone dans la partie alkoxy, alkoximinoalkyle ayant 1 à 4 atomes de carbone dans la partie alkoxy et 1 à 3 atomes de carbone dans la partie alkyle, nitro et/ou cyano,

$R^2$ représente de l'hydrogène, un groupe alkyle ayant 1 à 6 atomes de carbone, alcényle ayant 3 à 6 atomes de carbone, acyle ayant 1 à 4 atomes de carbone ou phénylalkyle ayant 1 à 4 atomes de carbone dans la partie alkyle,

$X^1$ représente du fluor, du chlore, du brome ou de l'iode,

$X^2$ représente du fluor, du chlore, du brome ou de l'iode,

$X^3$ est de l'hydrogène, du chlore, du brome ou de l'iode et

Y est un atome d'azote ou un groupe CH,

ou aussi

$R^1$ est un groupe phényle qui peut être substitué une à trois fois identiques ou différentes par un halogène, un radical alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et 1 à 5 atomes d'halogènes identiques ou différents, halogénalkoxy ayant 1 ou 2 atomes de carbone et 1 à 5 atomes d'halogènes identiques ou différents, halogénalkylthio ayant 1 ou 2 atomes de carbone et 1 à 5 atomes d'halogènes identiques ou différents, cycloalkyle ayant 3 à 7 atomes de carbone, phényle, phénoxy, alkoxycarbonyle ayant 1 à 4 atomes de carbone dans la partie alkoxy, alkoximinoalkyle ayant 1 à 4 atomes de carbone dans la partie alkoxy et 1 à 4 atomes de carbone dans la partie alkyle, nitro et/ou cyano,

<u>dans la mesure où</u>

$X^3$ est de l'hydrogène, du brome ou de l'iode,

ainsi que de leurs sels d'addition d'acides et de leurs complexes de sels métalliques, caractérisé en ce que :

a) on fait réagir des alcynes de formule

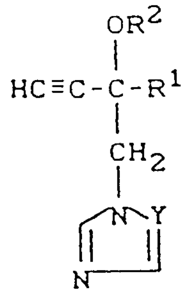

(II)

dans laquelle

$R^1$, $R^2$ et Y ont la définition indiquée ci-dessus,

avec du fluor, du chlore, du brome ou de l'iode ou avec des composés fournissant ces halogènes en présence d'un diluant,

ou bien

b) on fait réagir des alcènes de formule

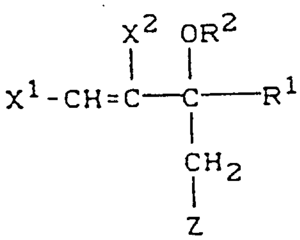

(III)

dans laquelle

$R^1$, $R^2$, $X^1$ et $X^2$ ont la définition indiquée ci-dessus et

Z représente le chlore, le brome, l'iode, un groupe méthylsulfonate ou p-tolylsulfonate,

avec des azoles de formule

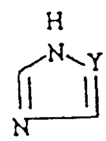

(IV)

dans laquelle

Y a la définition indiquée ci-dessus,

en présence d'un accepteur d'acide et en présence d'un diluant,

ou bien

c) on fait réagir des alcynes de formule

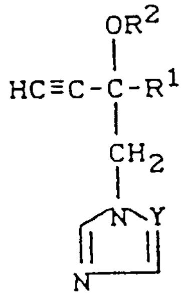

(II)

dans laquelle

$R^1$, $R^2$ et Y ont la définition indiquée ci-dessus,

dans une première étape, avec des hypohalogénites de formule

$MOX^4$ (V)

dans laquelle

M est un ion sodium ou potassium et

$X^4$ représente du chlore, du brome ou de l'iode,

en présence d'un diluant et, dans une seconde étape, on fait réagir les halogénalcynes ainsi obtenus de formule

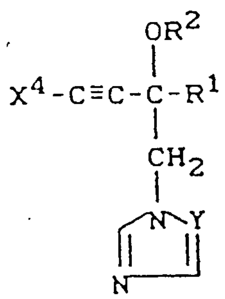

(VI)

dans laquelle

$R^1$, $R^2$, $X^4$ et Y ont la définition indiquée ci-dessus,

avec le fluor, le chlore, le brome ou l'iode ou avec des composés fournissant ces halogènes, en présence d'un diluant,

puis, le cas échéant, on additionne un acide ou un sel métallique sur les composés de formule (I) ainsi obtenus.

**2.** Microbicides, caractérisés par une teneur en au moins un dérivé halogénovinyl-azolique de formule (I) suivant la revendication 1 ou en un sel d'addition d'acide ou un complexe de sel métallique d'un dérivé halogénovinylazolique de formule (I).

**3.** Utilisation de dérivés halogénovinyl-azoliques de formule (I) suivant la revendication 1 ou de leurs sels d'addition d'acides et de leurs complexes de sels métalliques comme microbicides dans la protection des plantes et la protection de matériaux.

**4.** Procédé pour combattre des micro-organismes indésirables dans la protection des plantes et dans la protection de matériaux, caractérisé en ce qu'on fait agir des dérivés halogénovinyl-azoliques de formule (I) suivant la revendication 1 ou leurs sels d'addition d'acides ou leurs complexes de sels métalliques sur les micro-organismes et/ou leurs milieux.

**5.** Procédé de préparation de compositions microbicides, caractérisé en ce qu'on mélange des dérivés hydroxyéthyl-azoliques de formule (I) suivant la revendication 1 ou leurs sels d'addition d'acides ou leurs complexes de sels métalliques avec des diluants et/ou des agents tensio-actifs.